# EUROPÄISCHE PATENTSCHRIFT

(11) **EP 1 981 553 B1**
(45) Veröffentlichungstag und Bekanntmachung des Hinweises auf die Patenterteilung: **22.03.2017**
(21) Anmeldenummer: 07711497.3
(22) Anmeldetag: 09.02.2007
(51) Int. Cl.: A61L 24/04, A61L 24/02

(54) **KLEBER FÜR MEDIZINISCHE ANWENDUNGEN UND MITTEL ZUR BLUTSTILLUNG**
ADHESIVE FOR MEDICAL APPLICATIONS AND MEANS FOR HAEMOSTASIS
ADHÉSIF DESTINÉ À DES APPLICATIONS MÉDICALES ET AGENT HÉMOSTATIQUE

(30) Priorität: 09.02.2006 DE 102006006904
(43) Veröffentlichungstag der Anmeldung: 22.10.2008
(73) Patentinhaber: Stryker European Holdings I, LLC, Kalamazoo, MI 49002 (US)
(72) Erfinder: SPEITLING, Andreas, Werner, 24149 Kiel (DE); PROCTER, Philip, F-01220 Divonne les Bains (FR); SCHOMBURG, Joachim, 17034 Neubrandenburg (DE); SCHULTZ, Christian, 17033 Neubrandenburg (DE); JÜLICH, Wolf-Dieter, 17489 Greifswald (DE); LINDEQUIST, Ulrike, 17489 Greifswald (DE); SCHAUER, Frieder, 17509 Lubmin (DE); MIKOLASCH, Anett, 17489 Griefswald (DE); MANDA, Katrin, 18581 Putbus (DE)
(74) Vertreter: Maiwald Patentanwalts GmbH
(86) Internationale Anmeldenummer: PCT/EP2007/001131
(87) Internationale Veröffentlichungsnummer: WO 2007/090673

(56) Entgegenhaltungen:
- WO-A-00/69480
- WO-A-03/035122
- US-A- 4 542 115
- US-A- 5 015 677
- US-A1- 2003 187 387
- "Römpp Chemie Lexikon", 1992, Thieme Verlag, Stuttgart ISBN: 3-13-735109-X pages 5119-5121,
- "Linde Type A (LTA)" In: Ch. Baerlocher, W.M. Meier, D.H. Olson: "Atlas of Zeolite Framework Types", 2001, Elsevier

## Beschreibung

Die Erfindung betrifft Kleber für medizinische Anwendungen und Mittel zur Blutstillung. Insbesondere betrifft die Erfindung einen Kit zur Herstellung eines Klebers, der geeignet ist, Hart- und Weichgewebe miteinander zu verkleben, Wunden zu verschließen, sowie den Kleber selbst als auch dessen Verwendungen im medizinischen Bereich. Der Kit bzw. der dazugehörige Kleber umfasst als Einzelkomponenten Substrate der Polyphenoloxidase sowie Polyphenoloxidasen als solche. Darüber hinaus richtet sich die Erfindung auf den kombinierten Einsatz von Hämostyptika und Kleber beim Wundverschluss.

### Stand der Technik

Die Entwicklung neuer Gewebekleber, die insbesondere für Problemfälle eingesetzt werden können, hat in den letzten Jahren stark an Bedeutung gewonnen. Bisher eingesetzte Gewebekleber auf Acrylat-Basis können nur auf oberflächlichen Wunden eingesetzt werden, da die Acrylate biologisch nicht abgebaut werden und eine toxische Gefährdung bedeuten können. Ein Augenmerk liegt deshalb vor allem auf der biologischen Abbaubarkeit der eingesetzten Materialien. Aus diesem Grund spielen für die Klebstoffherstellung beispielsweise Polymere pflanzlichen bzw. tierischen Ursprungs eine große Rolle. Muscheln sind z. B. in der Lage, unter Wasser besonders feste Verbindungen mit verschiedenen Trägersubstraten einzugehen. Grund dafür ist die Bildung/Ausscheidung von MAPs (mussel adhesive proteins). MAPs bestehen aus einer Vielzahl von Proteinen, die je nach Muschelart variieren kann. Im US 5015 677 A wird ein Gewebekleber geschützt, der ein ganz spezielles aus Muscheln isoliertes Dekapeptid als essentielle Komponente nutzt. Dieses Dekapeptid enthält Dihydroxy-Aromaten als Substituenten von sehr speziellen Aminosäuren, die als essentieller Bestandteil der Peptid-Kette anzusehen sind. Die Peptid-Ketten werden dann unter dem Einfluss einer Tyrosinase miteinander verknüpft. Darüber hinaus sind keine befriedigenden Lösungen bekannt, die es ermöglichen Knochen mit Implantaten mittels Kleber zu fixieren.

EP 0 947 142 offenbart die Erhöhung des Molekulargewichts von Proteinen durch ihre Vernetzung durch Multi-Kupfer-Enzyme. Die beschriebene Erhöhung des Molekulargewichts der Substratproteine trat nach 17 Stunden Inkubation auf. Die mit Hilfe des Verfahrens nach EP 0 947 142 vernetzten Proteine sind vor allem zur Verwendung in Nahrungsmitteln geeignet, z.B. zur Veränderung der Konsistenz von Wurst. Eine Kreuzvernetzung von Proteinen durch substituierte Dihydroxyaromaten wird in EP 0 947 142 nicht offenbart.

Wenn Wundkleber bei stark blutenden Wunden eingesetzt werden sollen, besteht die Gefahr, dass Klebekomponenten mit dem Blutstrom weggespült werden und die Verklebung an einem unerwünschten Ort eintritt. Damit wird die Thrombose-Gefahr stark erhöht. Die akute Versorgung stark blutender Wunden ist auch heute noch ein unbefriedigend gelöstes Problem. Eine Blutstillung kann nach dem Stand der Technik erreicht werden durch
a) Proteindenaturierung z.B. durch Metallsalze
b) mikroporöse Biopolymere bzw. Biopolymere mit großer innerer Oberfläche. Diese absorbieren Wasser, dadurch kommt es zu einer Konzentration der Blutzellen sowie der Gerinnungsfaktoren. Als besonders vorteilhaft haben sich Polysaccharide erwiesen, die aus Poly-N-Acetyl-Glucosaminen oder Chitosan bestehen. Diese sind z.B. im Skelett von Insekten, aber auch in Algen enthalten. Biopolymere wie Gelatine, Collagen, Fibrinschwämme oder oxidierte Cellulose haben eine große innere Oberfläche. Aufgrund der Absorption an diese große Fläche kommt es zu einer Konzentration der Gerinnungsfaktoren.
d) hochkonzentrierte Gerinnungsfaktoren
e) Kombinationen von c) und d)
f) Calzium-Alginate
g) alumosilikatische Syntheseprodukte mit Mikroporenstruktur.

Die bisher zur Blutstillung eingesetzten alumosilikatische Syntheseprodukte sind Alkali- und Erdalkalialuminiumsilicate unterschiedlicher Zusammensetzung.

Auf der Basis von synthetisierten und bei über 400 °C vollständig dehydratisierten Erdalkalialuminiumsilicaten wurde von der Firma Z-Medica, U.S. für die Sofortbehandlung von Kriegsverletzungen und zur Notversorgung ein Syntheseprodukt einwickelt. Durch eine starke exotherme Reaktion wird dem Blut Wasser entzogen und es kommt so zur Blutstillung. Das Syntheseprodukt wird direkt in Granulatform auf die Wunde appliziert. Es wird unter der Bezeichnung "Quikclot" vertrieben. Nachteilig wurde bei diesem Produkt beschrieben, dass durch die exothermen Reaktion (Temperaturen bis 60 °C) Schädigungen des angrenzenden Gewebes auftreten. (Journal of Trauma 54 (2003) 6, 1077 - 1082).

Synthetische Lithiumalumosilikate (EP 1176991 A1, WO 00/69480) wurden speziell für die Wundversorgung entwickelt und werden unter dem Handelsnamen CERDAC vertrieben. Im Falle von CERDAC wird unter Aufwendung hoher Temperaturen (> 1000 °C) ein mikroporosiertes keramisches Produkt hergestellt, um eine optimale Wundversorgung zu erreichen.

Abgesehen von der z.T. aufwändigen Herstellung ist die Kapillarkraftwirkung zu gering, um allen Anforderungen zu genügen.

US 4,542,115 beschreibt ein Zeolithgranulat und ein Verfahren zu dessen Herstellung. Eine Verwendung dieses Granulats zur Wundversorgung wird nicht beschrieben.

WO 03/035122 beschreibt eine Zusammensetzung zum Verkleben von biologischem Gewebe, umfassend eine wässrige Lösung mindestens eines aminogruppentragenden Polymers und eine wässrige Lösung mindestens eines Aldehyds mit mindestens 3 Aldehydgruppen, wobei die Zusammensetzung frei von Eiweiß ist.

Ein großes Problem besteht weiterhin in einer möglichen Chronifizierung der Wunden, insbesondere bei Risikopatienten. Diese kann bisher nicht sicher vermieden werden, und die Behandlung solcher Wunden ist nach wie vor sehr schwierig. Mit der Chronifizierung ist oft eine Keimbesiedlung der Wunde verbunden.

Allein in Deutschland leiden 4 Millionen Patienten an chronisch offenen Wunden. Die jährlichen Kosten betragen 1,7 bis 3,2 Milliarden Euro. Für die Versorgung chronischer Wunden wurde bisher keine befriedigende Lösung erreicht.

Insgesamt kann festgestellt werden, dass sowohl für das Problem der Verklebung von Hart- und Weichteilgewebe als auch eine schnelle Verschließung, die Versorgung von stark blutenden als auch vor allem für die Versorgung chronischer Wunden bisher noch keine in jeder Hinsicht befriedigenden Lösungen erreicht wurden.

Die vorliegende Aufgabe besteht nun darin, die im Stand der Technik aufgeführten Probleme durch Produkte, die effiziente Klebstoffeigenschaften für Hart- und Weichteilgewebe aufweisen, zu lösen. Insbesondere sollten Systeme, wie Klebstoffe, bereitgestellt werden, die es ermöglichen stark blutende Wunden zu stillen und / oder Knochenpartien miteinander oder Knochenpartien mit Implantaten fest zu verbinden. Wünschenswert wären auch Klebstoffe, die es ermöglichen, Weichgewebe mit Knochen zu verbinden, wie bei Knorpeltransplantaten. Die gefundenen Möglichkeiten für einen Wundverschluss sollten vorzugsweise auch auf chronische und infizierte Wunden übertragbar sein.

Die Erkenntnis der vorliegenden Erfindung ist, dass Polymere, die mindestens eine freie Aminogruppe aufweisen, mittels Brückenmolekülen unter Einsatz von (lignolytischen) Polyphenoloxidasen unter Ausbildung kovalenter Bindungen untereinander und / oder mit körpereigenem Gewebe vernetzt werden und somit als Hart- und Weichteilgewebekleber eingesetzt werden können.

Eine weitere Erkenntnis der vorliegenden Erfindung ist, dass spezifisch aufbereitete Naturzeolithe eine rasche Blutstillung bewirken.

Insofern richtet sich die vorliegende Erfindung in einer ersten Ausführungsform auf ein Kit, welches die folgenden Einzelkomponenten umfasst:
a) Polymere mit mindestens einer freien Aminogruppe,
b) Brückenmoleküle ausgewählt aus der Gruppe bestehend aus monocyclischen ortho-Dihydroxyaromaten, monocyclischen para-Dihydroxyaromaten, bicyclischen Monohydroxyaromaten, polycyclischen Monohydroxyaromaten, bicyclischen Dihydroxyaromaten, polycyclischen Dihydroxyaromaten, bicyclischen Trihydroxyaromaten, polycyclischen Trihydroxyaromaten, und deren Mischungen, und
c) Polyphenoloxidasen , insbesondere lignolytische Polyphenoloxidasen,
wobei die beiden Einzelkomponenten b) und c), d.h. die Brückenmoleküle mit den Polyphenoloxidasen, insbesondere lignolytische Polyphenoloxidasen, nicht in Kontakt stehen.

Unter einem Kit wird in der vorliegenden Erfindung insbesondere eine Verpackung verstanden, die es ermöglicht die Einzelkomponenten so nebeneinander aufzubewahren, dass sie nicht in Kontakt stehen. Im vorliegenden Fall müssen zumindest die Brückenmoleküle (Komponente b)) von den (lignolytischen) Polyphenoloxidasen (Komponente c)) getrennt sein, damit eine frühzeitige und unerwünschte Reaktion vermieden wird. Folglich ist eine Mischung der Komponenten a) und b) und / oder der Komponenten a) und c) möglich.

Um einen Kontakt zwischen den Brückenmolekülen und den (lignolytischen) Polyphenoloxidasen zu vermeiden, können beide Einzelkomponenten vorzugsweise in unterschiedlichen Gefäßen oder Kammern aufbewahrt werden. Dies gilt auch für den Fall, falls eine oder beide Einzelkomponenten b) und c) mit dem Polymer a) vermischt ist. Denkbar ist eine Spritze, die es ermöglicht, über zwei Kanülen die beiden Einzelkomponenten, d.h. die Brückenmoleküle und die (lignolytischen) Polyphenoloxidasen, getrennt voneinander abzugeben. Dabei wird bevorzugt, dass die beiden Einzelkomponenten, d.h. die Brückenmoleküle und die (lignolytischen) Polyphenoloxidasen, in der Spritze in unterschiedlichen Kammern vorliegen. Denkbar ist natürlich auch, dass der Kit mindestens zwei Spritzen umfasst, wobei eine die Brückenmoleküle enthält und die andere die (lignolytischen) Polyphenoloxidasen.

In einer Ausführungsform stellt der Kit eine Zweikomponentenspritze dar, bevorzugt bereits mit angesetztem Mischextruder. Dies erlaubt eine besonders exakte Dosierung und einfache Handhabung. Bevorzugt ist eine Doppelkammerspritze etwa vom Typ Mixpac (Mixpac Systems AG, Rotkreuz, Schweiz).

Es ist selbstverständlich, dass im Rahmen der Erfindung unabhängig voneinander ein oder mehrere verschiedene Einzelkomponenten a), b) und c) eingesetzt werden können. Einzahl und Mehrzahl wird daher in Bezug auf alle Komponenten synonym gebraucht.

Eine zwingende Voraussetzung ist, dass der Kit als Einzelkomponente a) Polymere umfasst, die mindestens eine freie Aminogruppe enthalten. Unter freier Aminogruppe wird in vorliegender Erfindung eine Aminogruppe verstanden, die nicht Bestandteil der Polymerkette ist, sondern einen Rest der Polymerkette darstellt. Nachdem diese Polymere in dem Kit vorliegen sind diese nicht Bestandteil des menschlichen Körpers. Bei den Polymeren handelt es sich vorzugsweise um Polymere, die mehr als eine Aminogruppe umfassen, d.h. um aminogruppenreiche Polymere, wie Peptide.

Bei den Peptiden (d.h. bei Oligopeptiden und Proteinen) besteht naturgemäß eine Amidbindung zwischen den Aminosäuren. Vorzugsweise sind die Peptide Lysinhaltig. Gemäß einer bevorzugten Ausführungsform der Erfindung werden Lysin-haltige Oligopeptide verwendet. Denkbar ist auch, dass das Peptid Kollagen ist.

Ist das Polymer ein Peptid ergibt sich als besonderer Vorteil, dass die durch Reaktion der Komponenten entstandene Verbindung biologisch abbaubar ist. Durch die Verknüpfung von Peptidsequenzen, die die für eine Klebewirkung relevanten Aminosäuren enthalten, entsteht eine sehr feste Verklebung, die aber im Verlauf des Heilungsprozesses resorbiert werden kann. Die anfängliche Härtungsphase, verbunden mit hohen Klebeigenschaften, und die zeitabhängig langsame Resorption im Körper bei fortgeschrittenen Heilungsprozessen sind von besonderem Vorteil, da in die Klebestelle zunehmend körpereigenes Gewebe eingelagert wird.

Im Rahmen der vorliegenden Erfindung wird als Peptid sowohl ein Oligopeptid (2 bis 100 Aminosäuren Länge, bevorzugt 4 bis 20 Aminosäuren Länge oder 6 bis 10 Aminosäuren Länge) als auch ein Protein (Eiweiß, 100 bis 5000 Aminosäuren Länge, bevorzugt 100-1000 oder 100-200 Aminosäuren Länge) bezeichnet. Bevorzugt weist das Peptid eine Länge von 10-1000 Aminosäuren auf. Das Peptid kann ein Molekulargewicht von 1 bis 100 oder bis 200 kDa, insbesondere 2 bis 50 kDa oder 5 bis 20 kDa aufweisen. Es kann modifiziert bzw. substituiert sein, z.B. glykosyliert. Neben den üblichen proteinogenen Aminosäuren können auch modifizierte bzw. untypische Aminosäuren wie Hydoxylysin in dem Oligopeptid enthalten sein. Der Einsatz von D-Aminosäuren anstelle von L-Aminosäuren oder zusätzlich dazu ist möglich und verlangsamt den Abbau des Peptids.

Die Aminogruppen können vorzugsweise primäre oder sekundäre Aminogruppen sein. Besonders reaktiv sind jedoch primäre Aminogruppen. Mindestens eine der reaktiven Gruppen des Peptids ist bevorzugt Bestandteil einer Diaminosäure, z.B. von Lysin. Bevorzugt umfasst das Peptid daher mindestens eine Diaminosäure, bevorzugt mindestens 2, 3, 4, 5 oder mehr Diaminosäuren. Bevorzugt werden, wie oben erwähnt, Lysin-haltige Peptide (Oligopeptide oder Eiweiße) eingesetzt.

Auch andere Aminosäuren, wie Arginin, Asparagin, Glutamin oder Histidin, tragen reaktive Aminogruppen, die mit dem Dihydroxyaromaten reagieren können.

Die Aminogruppen, insbesondere die durch die Diaminosäure bereitgestellten Aminogruppen, sind für die Vernetzungsreaktion zwischen Peptid und Brückenmolekül besonders geeignet. Auch Hydroxylgruppen oder Merkaptogruppen in dem Peptid können jedoch zu der Vernetzungsreaktion beitragen. Bevorzugt umfasst das Peptid daher mindestens eine Aminosäure mit einer Hydroxylgruppe, also insbesondere Serin, Threonin oder Tyrosin oder einer Merkaptogruppe, z.B. Cystein. Auch Hydroxylysin oder polyphenolische Aminosäurebausteine, wie sie in den MAPs vorliegen, können in den erfindungsgemäß eingesetzten Peptiden vorkommen. Ein Vorteil der vorliegenden Erfindung ist jedoch, dass die Anwesenheit dieser spezifischen Aminosäurebausteine und damit die Verwendung von MAPs nicht zwingend notwendig ist. Daher ist in einer bevorzugten Ausführungsform das Polymer frei von polyphenolischen Aminobausteinen, d.h. frei von MAPs. Die verwendeten Peptide können daher z.B. ohne weiteres rekombinant hergestellt werden.

Die durch Reaktion der Brückenmoleküle mit dem Peptid entstehende Vernetzung hängt auch davon ab, wie hoch der Anteil der zur Verfügung stehenden reaktiven Gruppen in dem Peptid ist. Gute Klebeeigenschaften können ab einem Anteil an Aminosäuren des Peptids, die eine reaktive Aminogruppe (z.B. Diaminosäuren wie Lysin) tragen, von mindestens 10 % erreicht werden. Ist das Polymer kein Peptid, so beträgt der Anteil an Polymerstrukturelementen, die eine Aminogruppe tragen, vorzugsweise mindestens etwa 10%. Noch mehr bevorzugt liegt der Anteil dieser Aminosäuren oder Strukturelemente jedoch höher, bei mindestens 20%, mindestens 30%, mindestens 40% oder mindestens 50% oder sogar bei mindestens 80 bis 100%. Natürlich vorkommende Peptide und Proteine, z.B. Albumin oder Casein, können verwendet werden, besonders geeignet sind z.B. MAPs.

Es können jedoch auch weitere kürzere, leicht künstlich herstellbare Peptide eingesetzt werden. In einer besonders bevorzugten Ausführungsform der Erfindung sind etwa 50% der Aminosäuren des Peptids Lysin. Lysin und eine weitere Aminosäure können z.B. als sich wiederholende Dipeptid-Einheit angeordnet sein. Auch eine andere Abfolge oder die Aufnahme weiterer Aminosäuren, insbesondere von Arginin, Asparagin, Glutamin oder Histidin (anstelle von Lysin oder zusätzlich), Serin oder Threonin (anstelle von Tyrosin oder zusätzlich), von Cystein oder anderen Aminosäuren ist jedoch möglich. Eine Länge des Peptids von 10-20 Aminosäuren oder eine Mischung von Peptiden verschiedener Kettenlänge ist besonders bevorzugt.

In einer besonders bevorzugten Ausführungsform handelt es sich um ausschließlich aus zwei Aminosäuren bestehende Polymere, beispielsweise (Lysin-Tyrosin)ₙ, wobei n = Werte zwischen 5 und 40, wie 5, 10 oder 20, annehmen kann.

Eine weitere zwingende Voraussetzung der vorliegenden Erfindung ist, dass der Kit Brückenmoleküle umfasst, die eine Vernetzung von Polymeren, die mindestens eine freie Aminogruppe (wie Diaminosäuren) aufweisen, wie weiter oben definiert, bewirken.

Als Brückenmoleküle werden monocyclische ortho-Dihydroxyaromaten, monocyclische para-Dihydroxyaromaten, bicyclische Monohydroxyaromaten, polycyclische Monohydroxyaromaten, bicyclische Dihydroxyaromaten, polycyclische Dihydroxyaromaten, bicyclische Trihydroxyaromaten, polycyclische Trihydroxyaromaten, oder deren Mischungen eingesetzt. Die erfindungsgemäßen Hydroxyaromaten sind im Gegensatz zu den bekannten Muschelklebstoffen nicht Bestandteil einer Polymerkette.

Denkbar ist auch, dass diese Aromaten weiter substituiert sind. Bevorzugte funktionelle Gruppen sind Substituenten ausgewählt aus der Gruppe bestehend aus Halogen, Sulfo-, Sulfon-, Sulfamido-, Sulfanyl-, Amino-, Amido-, Azo-, Imino- und Hydroxy. Dabei wird insbesondere beobachtet, dass substituierte Aromaten, insbesondere substituierte Dihydroxyaromaten, überraschenderweise besonders günstige Polymerisationseigenschaften aufweisen, insbesondere wird eine schnelle Polymerisation, eine geringere Eigenkopplung und eine gute Festigkeit der Verklebung erreicht. Eine geeignete Substitution der Aromaten kann sogar dazu führen, dass nunmehr als Brückenmolekül auch Monohydroxyaromaten zur Vernetzung geeignet sind. Substituiert bedeutet im Rahmen dieser Erfindung insbesondere, dass an den Aromaten neben den Hydroxylgruppen noch 1, 2, 3 oder 4 weitere Reste gebunden sind. Andererseits sind auch monohydroxylierte Biarylverbindungen geeignet.

Besonders bevorzugt sind Phenolderivate, die eine Hydroxygruppe oder eine Methoxygruppe an der ortho- oder para-Position aufweisen. Folglich sind folgende Verbindungen der Formel 3 und 4 bevorzugt: 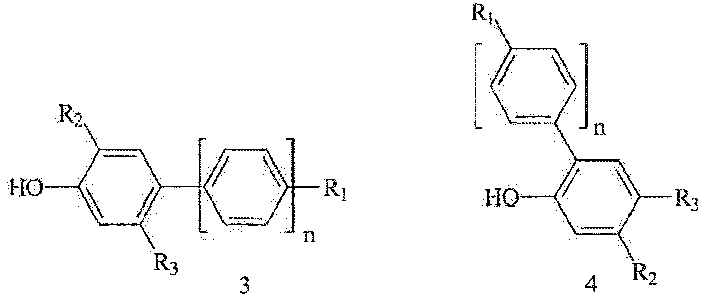 wobei
n = 0 - 10, bevorzugt 0 oder 1, insbesondere 0,
R₁ = OH oder NH₂ oder Hal, bevorzugt OH, Cl oder Br, insbesondere OH,
R₂ = H, CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und R₃ = H, CH₃, Alkyl, substituierter Aromat, insbesondere H oder CH₃, ist.

Alkyl bedeutet verzweigte oder unverzweigte aliphatische Kohlenwasserstoffketten, bevorzugt mit 1-20, mehr bevorzugt 1-6 Kohlenstoffen, z.B. Methyl, Ethyl, Propyl, Butyl, Isobutyl, n-Pentyl, n-Hexyl.

Möglich sind Verbindungen der Formel 3 und davon das Hydrochinon, welches weiter substituiert sein kann. Unter dem Aspekt einer möglichst schnellen Klebereaktion sind substituierte Dihydroxyaromaten mit geringer Eigenkopplung erfindungsgemäß besonders geeignet. Bevorzugt werden 2,5-Dihydroxybenzamide eingesetzt, besonders bevorzugt wird 2,5-Dihydroxy-N-(2-hydroxyethyl)-benzamid.

Im Fall der Trihydroxyaromaten ist es bevorzugt, dass nicht mehr als zwei Hydoxygruppen pro Benzoleinheit vorliegen. Besonders bevorzugt sind Polyphenyle, d.h. Biphenyl oder Triphenyl, folgender Formel 5: 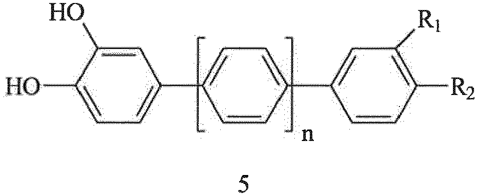 wobei
n = 0 - 10, bevorzugt 0 oder 1
und
R₁ = H und R₂ = OH oder R₁ = OH und R₂ = H
ist.

Dabei können die Phenyle der Formel 5 substituiert sein, z.B. in ortho-Position zu einer OH-Gruppe mit CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und / oder in meta-Position zu einer OH-Gruppe mit CH₃, Alkyl, substituierter Aromat, insbesondere CH₃.

Des Weiteren ist erforderlich, dass der Kit als weitere Einzelkomponente Polyphenoloxidasen, wie lignolytische Polyphenoloxidasen, insbesondere Laccase (EC 1.10.3.2), umfasst. Laccasen sind im Stand der Technik bekannt. Sie können aus Pflanzen, Pilzen, Bakterien oder Insekten stammen oder von natürlichen Enzymen abgeleitet sein. Die im Rahmen der Erfindung verwendeten Laccasen können rekombinant hergestellt oder aufgereinigt sein. Dabei ist eine besondere Reinheit der Laccase im allgemeinen nicht erforderlich, gegebenenfalls können auch Überstände lignolytischer Pilze eingesetzt werden. Für medizinische Anwendungen ist jedoch eine wesentliche Abtrennung von mikrobiologischen Substanzen wie Lipopolysacchariden oder anderen Zellwandbestandteilen oft wünschenswert. Beispiele sind Laccase aus den Gattungen *Aspergillus, Neurospora, Podospora, Botrytis, Collybia, Fomes, Lentinus, Pleurotus, Pycnoporus, Pyricularia, Trametes, Rhizoctonia, Coprinus, Psatyrella, Myceliophthora, Schtalidium, Polyporus, Phlebia* oder *Coriolus.* Die Herstellung von Laccasen ist z.B. in EP 0 947 142 offenbart.

Durch den Einsatz von Polyphenoloxidasen, wie lignolytischen Polyphenoloxidasen, vorzugsweise Laccase (EC 1.10.3.2) im Kit kann deren breites sowie besonderes Substratspektrum für die Klebereaktion ausgenutzt werden. Daher zeichnet sich der Kit insbesondere dadurch aus, dass die Klebereaktion nicht auf spezielle in der Natur gefundene Peptide, die als Brückenmolekül fungieren, beschränkt ist, sondern ein weites Spektrum von Brückenmolekülen, einerseits, sowie von Peptiden, wie Oligopeptiden, oder Proteinen, andererseits, eingesetzt werden kann.

Variationen der Mengenverhältnisse sind sowohl bei der Einzelkomponente a), insbesondere dem Peptid, als auch beim Brückenmolekül etwa von 1-50 mM möglich. Für jede Anwendung ist das optimale Mengenverhältnis in Vorversuchen zu ermitteln.

Dabei muss beachtet werden, dass je nach dem ausgewählten Brückenmolekül auch eine Eigenreaktion des Brückenmoleküls stattfindet, die die Bildung von Vernetzungsprodukten mindert. Zu geringe Konzentrationen der Brückenmoleküle führen zu einer zu langsamen Reaktion, zu hohe Konzentrationen zu stärkeren Nebenreaktionen durch Eigenkopplung. Wenn ein geringerer Anteil an Peptid vorliegt, werden reaktive Amino-, Merkapto- und/oder Hydroxylgruppen von zu verklebenden Substraten verstärkt in die Vernetzung einbezogen. Die Menge an Polyphenoloxidase beeinflusst die Geschwindigkeit der Rektion, wobei, je nach Anwendung, ein schnelles Erreichen des Gelpunkts oder vollständiges Aushärten oder eine längere Verarbeitbarkeit der Kombination erreicht werden kann. Durch die in Vorversuchen durchzuführende Optimierung der Mengeverhältnisse kann daher der Kit an die jeweilige konkrete Aufgabenstellung angepasst werden.

Es ist ein besonderer Vorteil des Kit, dass die Bestandteile im Kit in einem vorher optimierten Mengenverhältnis vorliegen, das für die jeweilige Verwendung angemessen ist. Eine bevorzugtes Mengenverhältnis für das Verkleben von Weichteilgeweben ist beispielsweise [Tyr-Lys]ₙ, n=4-35, 8,5 mM; 2,5-Dihydroxy-N-(2-hydroxyethyl)-benzamid 12,5 mM; Polyphenoloxidase: 0,32 U (156 nmol mol⁻¹ min⁻¹). Dieses Mengenverhältnis kann im Kit vorgegeben werden und erspart eine umständliche Einzeldosierung der Zutaten.

Die im Kit eingesetzte Kombination kann weitere Zusatz- und Hilfsstoffe enthalten, z.B. Füllmittel wie Collagen, Albumin, Hyaluronsäure oder ähnliches. Bevorzugt beträgt der Gesamtanteil von Einzelkomponente a), wie Peptid, und Brückenmolekül etwa 50-90%.

Die essentiellen Bestandteile des Kit werden bevorzugt mit einem oder mehreren wässrigen Lösungsmitteln gelöst. Für medizinische Anwendungen ist das Lösungsmittel nicht toxisch und biologisch verträglich. Bevorzugt ist als Lösungsmittel für biologische Anwendungen ein Phosphatpuffer wie Calciumphosphat- oder Natriumphosphatpuffer oder PBS. Für andere Anwendungen kann es aber auch ein organisches Lösungsmittel wie DMSO oder eine Mischung aus einem wässrigen und einem organischen Lösungsmittel sein. Alternativ kann die Kombination oder ihre Bestandteile erst vor der Verwendung in dem Lösungsmittel aufgenommen werden und in den Kit eingeführt werden.

Die Konsistenz der eingesetzten Komponenten im Kit muss jedoch nicht flüssig sein, sondern kann eine pastöse Konsistenz aufweisen. Die Viskosität und Fließfähigkeit der verwendeten Einzelkomponenten kann, abhängig z.B. von der Länge der zu fixierenden Wunde und der Wundspalttiefe bzw. den zu verklebenden Substraten angepasst werden. Neben der Länge der verwendeten Peptide/Polymere hat auch die Menge an Lösungsmittel einen Einfluss auf diese Parameter. Auch Zusatzstoffe wie Thixotropiemittel können zur Anpassung von Viskosität und Fließfähigkeit verwendet werden. Typischerweise wird zum Kleben von Hartgewebe eine Kombination mit höherer Viskosität verwendet als zum Kleben von Weichgewebe.

Der pH ist bevorzugt 2-10, insbesondere 5-7. Die Reaktion kann bei 2-80°C ablaufen, bevorzugt ist jedoch eine Temperatur von 20-37°C oder 25-30°C. Damit kann die Klebereaktion mittels Kit bei Raumtemperatur/Körpertemperatur durchgeführt werden.

Besondere Anforderungen an die Lagerung wie bei tiefgekühlten Fibrinklebern bestehen nicht. Die (lignolytische) Polyphenoloxidase, wie Laccase, kann in gelöster Form eingesetzt werden, wobei eine Lagerung bei Kühlschranktemperatur ausreichend ist. Es ist auch möglich, die (lignolytische) Polyphenoloxidase, wie Laccase, in Pulverform zu liefern und vor einer absehbaren Anwendung zu lösen.

Eine Sterilisierung des Kits bzw. seiner einzelnen Komponenten kann vorteilhafterweise ohne Strukturveränderung erreicht werden. Möglich ist z.B. eine Sterilfiltrierung von Lösungen. Bevorzugt ist jedoch eine Sterilisierung über Gamma-Strahlung, da diese bereits verpackt erfolgen kann und somit keine aseptische Abfüllung nötig ist. Der Aktivitätsverlust der (lignolytischen) Polyphenoloxidase, wie Laccase, durch die Gammasterilisation, der bis zu 50 % betragen kann, kann durch eine entsprechend höhere Ausgangskonzentration leicht kompensiert werden.

Vorzugsweise wird der oben definierte Kit als Medikament verwendet. Eine Klassifizierung als pharmazeutisches Präparat oder als Medizinprodukt ist, abhängig von nationalem Recht, ebenfalls möglich. Diese Begriffe sind für die Zwecke der Beschreibung der Erfindung austauschbar.

In einen weiteren Aspekt betrifft die vorliegende Erfindung einen Kleber mit den oben beschriebenen Einzelkomponenten. Folglich umfasst der Kleber die folgenden Einzelkomponenten:
a) Polymere mit mindestens einer freien Aminogruppe, wobei die Polymere nicht Bestandteil des menschlichen Körpers sind,
b) Brückenmoleküle ausgewählt aus der Gruppe bestehend aus monocyclischen ortho-Dihydroxyaromaten, monocyclischen para-Dihydroxyaromaten, bicyclischen Monohydroxyaromaten, polycyclischen Monohydroxyaromaten, bicyclischen Dihydroxyaromaten, polycyclischen Dihydroxyaromaten, bicyclischen Trihydroxyaromaten, polycyclischen Trihydroxyaromaten, und deren Mischungen, und
c) Polyphenoloxidasen, insbesondere lignolytische Polyphenoloxidasen,
wobei die beiden Einzelkomponenten b) und c), d.h. die Brückenmoleküle mit den Polyphenoloxidasen, insbesondere den lignolytische Polyphenoloxidasen, in Kontakt stehen, d.h. miteinander vermischt sind.

Bezüglich der einzelnen Komponenten wird auf die Ausführungen bezüglich dem Kit verwiesen.
Demnach ist es selbstverständlich, dass im Rahmen der Erfindung unabhängig voneinander ein oder mehrere verschiedene Einzelkomponenten a), b) und c) eingesetzt werden können. Einzahl und Mehrzahl wird daher in Bezug auf alle Komponenten synonym gebraucht.

Eine zwingende Voraussetzung ist, dass der Kleber als Einzelkomponente a) Polymere umfasst, die mindestens eine freie Aminogruppe enthalten. Unter freier Aminogruppe wird in vorliegender Erfindung eine Aminogruppe verstanden, die nicht Bestandteil der Polymerkette ist, sondern einen Rest der Polymerkette darstellt. Bei den Polymeren des Klebers handelt es sich um Polymere die nicht Bestandteil des menschlichen Körpers sind. Vorzugsweise handelt es sich bei den Polymeren um Polymere, die mehr als eine Aminogruppe umfassen, d.h. um aminogruppenreiche Polymere, wie Peptide.

Bei den Peptiden (d.h. bei Oligopeptiden und Proteinen) besteht naturgemäß eine Amidbindung zwischen den Aminosäuren. Vorzugsweise sind die Peptide Lysinhaltig. Gemäß einer bevorzugten Ausführungsform der Erfindung werden Lysin-haltige Oligopeptide verwendet. Denkbar ist auch, dass das Peptid Kollagen ist.

Ist das Polymer ein Peptid ergibt sich als besonderer Vorteil, dass die durch Reaktion der Komponenten entstandene Verbindung biologisch abbaubar ist. Durch die Verknüpfung von Peptidsequenzen, die die für eine Klebewirkung relevanten Aminosäuren enthalten, entsteht eine sehr feste Verklebung, die aber im Verlauf des Heilungsprozesses resorbiert werden kann. Die anfängliche Härtungsphase, verbunden mit hohen Klebeigenschaften, und die zeitabhängig langsame Resorption im Körper bei fortgeschrittenen Heilungsprozessen sind von besonderem Vorteil, da in die Klebestelle zunehmend körpereigenes Gewebe eingelagert wird.

Im Rahmen der vorliegenden Erfindung wird als Peptid sowohl ein Oligopeptid (2 bis 100 Aminosäuren Länge, bevorzugt 4 bis ca. 20 Aminosäuren Länge oder 6 bis 10 Aminosäuren Länge) als auch ein Protein (Eiweiß, 100 bis 5000 Aminosäuren Länge, bevorzugt 100-1000 oder 100-200 Aminosäuren Länge) bezeichnet. Bevorzugt weist das Peptid eine Länge von 10-1000 Aminosäuren auf. Das Peptid kann ein Molekulargewicht von 1 bis 100 oder bis 200 kDa, insbesondere 2 bis 50 kDa oder 5 bis 20 kDa aufweisen. Es kann modifiziert bzw. substituiert sein, z.B. glykosyliert. Neben den üblichen proteinogenen Aminosäuren können auch modifizierte bzw. untypische Aminosäuren wie Hydoxylysin in dem Oligopeptid enthalten sein. Der Einsatz von D-Aminosäuren anstelle von L-Aminosäuren oder zusätzlich dazu ist möglich und verlangsamt den Abbau des Peptids.

Die Aminogruppen können vorzugsweise primäre oder sekundäre Aminogruppen sein. Besonders reaktiv sind jedoch primäre Aminogruppen. Mindestens eine der reaktiven Gruppen des Peptids ist bevorzugt Bestandteil einer Diaminosäure, z.B. von Lysin. Bevorzugt umfasst das Peptid daher mindestens eine Diaminosäure, bevorzugt mindestens 2, 3, 4, 5 oder mehr Diaminosäuren. Bevorzugt werden, wie oben erwähnt, Lysin-haltige Peptide (Oligopeptide oder Eiweiße) eingesetzt.

Auch andere Aminosäuren, wie Arginin, Asparagin, Glutamin oder Histidin, tragen reaktive Aminogruppen, die mit dem Dihydroxyaromaten reagieren können.

Die Aminogruppen, insbesondere die durch die Diaminosäure bereitgestellten Aminogruppen, sind für die Vernetzungsreaktion zwischen Peptid und Brückenmolekül besonders geeignet. Auch Hydroxylgruppen oder Merkaptogruppen in dem Peptid können jedoch zu der Vernetzungsreaktion beitragen. Bevorzugt umfasst das Peptid daher mindestens eine Aminosäure mit einer Hydroxylgruppe, also insbesondere Serin, Threonin oder Tyrosin oder einer Merkaptogruppe, z.B. Cystein. Auch Hydroxylysin oder polyphenolische Aminosäurebausteine, wie sie in den MAPs vorliegen, können in den erfindungsgemäß eingesetzten Peptiden vorkommen. Ein Vorteil der vorliegenden Erfindung ist jedoch, dass die Anwesenheit dieser spezifischen Aminosäurebausteine und damit die Verwendung von MAPs nicht zwingend notwendig ist. Daher ist in einer bevorzugten Ausführungsform das Polymer frei von polyphenolischen Aminobausteinen, d.h. frei von MAPs. Die verwendeten Peptide können daher z.B. ohne weiteres rekombinant hergestellt werden.

Die durch Reaktion der Brückenmoleküle mit dem Peptid entstehende Vernetzung hängt auch davon ab, wie hoch der Anteil der zur Verfügung stehenden reaktiven Gruppen in dem Peptid ist. Gute Klebeeigenschaften können ab einem Anteil an Aminosäuren des Peptids, die eine reaktive Aminogruppe (z.B. Diaminosäuren wie Lysin) tragen, von mindestens 10 % erreicht werden. Ist das Polymer kein Peptid, so beträgt der Anteil an Polymerstrukturelementen, die eine Aminogruppe tragen, vorzugsweise mindestens etwa 10%. Noch mehr bevorzugt liegt der Anteil dieser Aminosäuren oder Strukturelemente jedoch höher, bei mindestens 20%, mindestens 30%, mindestens 40% oder mindestens 50% oder sogar bei mindestens 80 bis 100%. Natürlich vorkommende Peptide und Proteine, z.B. Albumin oder Casein, können verwendet werden, besonders geeignet sind z.B. MAPs.

Es können jedoch auch weitere kürzere, leicht künstlich herstellbare Peptide eingesetzt werden. In einer besonders bevorzugten Ausführungsform der Erfindung sind etwa 50% der Aminosäuren des Peptids Lysin. Lysin und eine weitere Aminosäure können z.B. als sich wiederholende Dipeptid-Einheit angeordnet sein. Auch eine andere Abfolge oder die Aufnahme weiterer Aminosäuren, insbesondere von Arginin, Asparagin, Glutamin oder Histidin (anstelle von Lysin oder zusätzlich), Serin oder Threonin (anstelle von Tyrosin oder zusätzlich), von Cystein oder anderen Aminosäuren ist jedoch möglich. Eine Länge des Peptids von 10-20 Aminosäuren oder eine Mischung von Peptiden verschiedener Kettenlänge ist besonders bevorzugt.

In einer besonders bevorzugten Ausführungsform handelt es sich um ausschließlich aus zwei Aminosäuren bestehende Polymere, beispielsweise (Lysin-Tyrosin)ₙ, wobei n = Werte zwischen 5 und 40, wie 5, 10 oder 20, annehmen kann.

Eine weitere zwingende Voraussetzung der vorliegenden Erfindung ist, dass der Kleber Brückenmoleküle umfasst, die eine Vernetzung von Polymeren, die mindestens eine freie Aminogruppe (wie Diaminosäuren) aufweisen, wie weiter oben definiert, bewirken.

Als Brückenmoleküle werden monocyclische ortho-Dihydroxyaromaten, monocyclische para-Dihydroxyaromaten, bicyclische Monohydroxyaromaten, polycyclische Monohydroxyaromaten, bicyclische Dihydroxyaromaten, polycyclische Dihydroxyaromaten, bicyclische Trihydroxyaromaten, polycyclische Trihydroxyaromaten, oder deren Mischungen eingesetzt. Die erfindungsgemäßen Hydroxyaromaten sind im Gegensatz zu den bekannten Muschelklebstoffen nicht Bestandteil einer Polymerkette.

Denkbar ist auch, dass diese Aromaten weiter substituiert sind. Bevorzugte funktionelle Gruppen sind Substituenten ausgewählt aus der Gruppe bestehend aus Halogen, Sulfo-, Sulfon-, Sulfamido-, Sulfanyl-, Amino-, Amido-, Azo-, Imino- und Hydroxy. Dabei wird insbesondere beobachtet, dass substituierte Aromaten, insbesondere substituierte Dihydroxyaromaten, überraschenderweise besonders günstige Polymerisationseigenschaften aufweisen, insbesondere wird eine schnelle Polymerisation, eine geringere Eigenkopplung und eine gute Festigkeit der Verklebung erreicht. Eine geeignete Substitution der Aromaten kann sogar dazu führen, dass nunmehr als Brückenmolekül auch Monohydroxyaromaten zur Vernetzung geeignet sind. Substituiert bedeutet im Rahmen dieser Erfindung insbesondere, dass an den Aromaten neben den Hydroxylgruppen noch 1, 2, 3 oder 4 weitere Reste gebunden sind. Andererseits sind auch monohydroxylierte Biarylverbindungen geeignet.

Besonders bevorzugt sind Phenolderivate, die eine Hydroxygruppe oder eine Methoxygruppe an der ortho- oder para-Position aufweisen. Folglich sind folgende Verbindungen der Formel 3 und 4 bevorzugt: 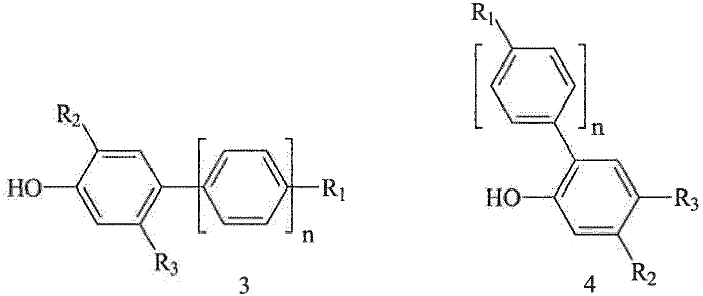 wobei
n = 0 - 10, bevorzugt 0 oder 1, insbesondere 0,
R₁ = OH oder NH₂ oder Hal, bevorzugt OH, Cl oder Br, insbesondere OH,
R₂ = H, CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und
R₃ = H, CH₃, Alkyl, substituierter Aromat, insbesondere H oder CH₃,
ist.

Alkyl bedeutet verzweigte oder unverzweigte aliphatische Kohlenwasserstoffketten, bevorzugt mit 1-20, mehr bevorzugt 1-6 Kohlenstoffen, z.B. Methyl, Ethyl, Propyl, Butyl, Isobutyl, n-Pentyl, n-Hexyl.

Möglich sind Verbindungen der Formel 3 und davon das Hydrochinon, welches weiter substituiert sein kann. Unter dem Aspekt einer möglichst schnellen Klebereaktion sind substituierte Dihydroxyaromaten mit geringer Eigenkopplung erfindungsgemäß besonders geeignet. Bevorzugt werden 2,5-Dihydroxybenzamide eingesetzt, besonders bevorzugt wird 2,5-Dihydroxy-N-(2-hydroxyethyl)-benzamid. Im Fall der Trihydroxyaromaten ist es bevorzugt, dass nicht mehr als zwei Hydoxygruppen pro Benzoleinheit vorliegen. Besonders bevorzugt sind Polyphenyle, d.h. Biphenyl oder Triphenyl, folgender Formel 5: 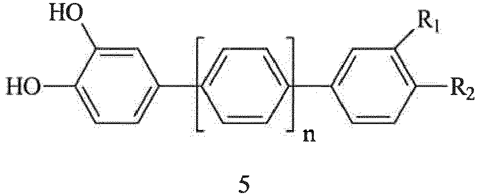 wobei
n = 0 - 10, bevorzugt 0 oder 1
und
R₁ = H und R₂ = OH oder R₁ = OH und R₂ = H
ist.

Dabei können die Phenyle der Formel 5 substituiert sein, z.B. in ortho-Position zu einer OH-Gruppe mit CH₃, CHO, COCH₃, CONH₂, CON-Alkyl, CON-Alkyl-OH, COOH, COO-Alkyl, Alkyl, substituierter Aromat, insbesondere CON-Alkyl oder COO-Alkyl, und / oder in metha-Position zu einer OH-Gruppe mit CH₃, Alkyl, substituierter Aromat, insbesondere CH₃.

Des Weiteren ist erforderlich, dass der Kleber als weitere Einzelkomponente Polyphenoloxidasen, wie lignolytische Polyphenoloxidasen, insbesondere Laccase (EC 1.10.3.2), umfasst. Laccasen sind im Stand der Technik bekannt. Sie können aus Pflanzen, Pilzen, Bakterien oder Insekten stammen oder von natürlichen Enzymen abgeleitet sein. Die im Rahmen der Erfindung verwendeten Laccasen können rekombinant hergestellt oder aufgereinigt sein. Dabei ist eine besondere Reinheit der Laccase im allgemeinen nicht erforderlich, gegebenenfalls können auch Überstände lignolytischer Pilze eingesetzt werden. Für medizinische Anwendungen ist jedoch eine wesentliche Abtrennung von mikrobiologischen Substanzen wie Lipopolysacchariden oder anderen Zellwandbestandteilen oft wünschenswert. Beispiele sind Laccase aus den Gattungen *Aspergillus, Neurospora, Podospora, Botrytis, Collybia, Fomes, Lentinus, Pleurotus, Pycnoporus, Pyricularia, Trametes, Rhizoctonia, Coprinus, Psatyrella, Myceliophthora, Schtalidium, Polyporus, Phlebia* oder *Coriolus.* Die Herstellung von Laccasen ist z.B. in EP 0 947 142 offenbart.

Durch den Einsatz von Polyphenoloxidasen, wie lignolytischen Polyphenoloxidasen, vorzugsweise Laccase (EC 1.10.3.2) im Kleber kann deren breites sowie besonderes Substratspektrum für die Klebereaktion ausgenutzt werden. Daher zeichnet sich der Kleber insbesondere dadurch aus, dass die Klebereaktion nicht auf spezielle in der Natur gefundene Peptide, die als Brückenmolekül fungieren, beschränkt ist, sondern ein weites Spektrum von Brückenmolekülen, einerseits, sowie von Peptiden, wie Oligopeptiden, oder Proteinen, andererseits, eingesetzt werden kann.

Variationen der Mengenverhältnisse sind sowohl bei der Einzelkomponente a), insbesondere dem Peptid, als auch beim Brückenmolekül etwa von 1-50 mM möglich. Für jede Anwendung ist das optimale Mengenverhältnis in Vorversuchen zu ermitteln.

Dabei muss beachtet werden, dass je nach dem ausgewählten Brückenmolekül auch eine Eigenreaktion des Brückenmoleküls stattfindet, die die Bildung von Vernetzungsprodukten mindert. Zu geringe Konzentrationen der Brückenmoleküle führen zu einer zu langsamen Reaktion, zu hohe Konzentrationen zu stärkeren Nebenreaktionen durch Eigenkopplung. Wenn ein geringerer Anteil an Peptid vorliegt, werden reaktive Amino-, Merkapto- und/oder Hydroxylgruppen von zu verklebenden Substraten verstärkt in die Vernetzung einbezogen. Die Menge an Polyphenoloxidase beeinflusst die Geschwindigkeit der Rektion, wobei, je nach Anwendung, ein schnelles Erreichen des Gelpunkts oder vollständiges Aushärten oder eine längere Verarbeitbarkeit der Kombination erreicht werden kann. Durch die in Vorversuchen durchzuführende Optimierung der Mengeverhältnisse kann daher der Kleber an die jeweilige konkrete Aufgabenstellung angepasst werden.

Es ist ein besonderer Vorteil des Klebers, dass die Bestandteile des Klebers in einem vorher optimierten Mengenverhältnis vorliegen, das für die jeweilige Verwendung angemessen ist. Eine bevorzugtes Mengenverhältnis für des Verkleben von Weichteilgeweben ist beispielsweise [Tyr-Lys]ₙ, n=4-35, 8,5 mM; 2,5-Dihydroxy-N-(2-hydroxyethyl)-benzamid 12,5 mM; Polyphenoloxidase: 0,32 U (156 nmol ml⁻¹ min⁻¹). Dieses Mengenverhältnis kann im Kleber vorgegeben werden und erspart eine umständliche Einzeldosierung der Zutaten.

Die im Kleber eingesetzte Kombination kann weitere Zusatz- und Hilfsstoffe enthalten, z.B. Füllmittel wie Collagen, Albumin, Hyaluronsäure oder ähnliches. Bevorzugt beträgt der Gesamtanteil von Einzelkomponente a), wie Peptid, und Brückenmolekül etwa 50-90%.

Die essentiellen Bestandteile des Klebers werden bevorzugt mit einem oder mehreren wässrigen Lösungsmitteln gelöst. Für medizinische Anwendungen ist das Lösungsmittel nicht toxisch und biologisch verträglich. Bevorzugt ist als Lösungsmittel für biologische Anwendungen ein Phosphatpuffer wie Calciumphosphat- oder Natriumphosphatpuffer oder PBS. Für andere Anwendungen kann es aber auch ein organisches Lösungsmittel wie DMSO oder eine Mischung aus einem wässrigen und einem organischen Lösungsmittel sein. Alternativ kann die Kombination oder ihre Bestandteile erst vor der Verwendung in dem Lösungsmittel aufgenommen werden.

Die Konsistenz der eingesetzten Einzelkomponenten des Klebers muss jedoch nicht flüssig sein, sondern kann eine pastöse Konsistenz aufweisen. Die Viskosität und Fließfähigkeit der verwendeten Einzelkomponenten und/oder des Klebers kann, abhängig z.B. von der Länge der zu fixierenden Wunde und der Wundspalttiefe bzw. den zu verklebenden Substraten angepasst werden. Neben der Länge der verwendeten Peptide/Polymere hat auch die Menge an Lösungsmittel einen Einfluss auf diese Parameter. Auch Zusatzstoffe wie Thixotropiemittel können zur Anpassung von Viskosität und Fließfähigkeit verwendet werden. Typischerweise wird zum Kleben von Hartgewebe eine Kombination mit höherer Viskosität verwendet als zum Kleben von Weichgewebe.

Der pH ist bevorzugt 2-10, insbesondere 5-7. Die Reaktion kann bei 2-80°C ablaufen, bevorzugt ist jedoch eine Temperatur von 20-37°C oder 25-30°C. Damit kann die Klebereaktion mittels Kleber bei Raumtemperatur/Körpertemperatur durchgeführt werden.

Besondere Anforderungen an die Lagerung wie bei tiefgekühlten Fibrinklebern bestehen nicht. Die (lignolytische) Polyphenoloxidase, wie Laccase, kann in gelöster Form eingesetzt werden, wobei eine Lagerung bei Kühlschranktemperatur ausreichend ist. Es ist auch möglich, die (lignolytische) Polyphenoloxidase, wie Laccase, in Pulverform zu liefern und vor einer absehbaren Anwendung zu lösen.

Eine Sterilisierung des Klebers bzw. seiner einzelnen Komponenten kann vorteilhafterweise ohne Strukturveränderung erreicht werden. Möglich ist z.B. eine Sterilfiltrierung von Lösungen. Bevorzugt ist jedoch eine Sterilisierung über Gamma-Strahlung, da diese bereits verpackt erfolgen kann und somit keine aseptische Abfüllung nötig ist. Der Aktivitätsverlust der (lignolytischen) Polyphenoloxidase, wie Laccase, durch die Gammasterilisation, der bis zu 50 % betragen kann, kann durch eine entsprechend höhere Ausgangskonzentration leicht kompensiert werden.

Vorzugsweise wird der oben definierte Kleber als Medikament verwendet. Eine Klassifizierung als pharmazeutisches Präparat oder als Medizinprodukt ist, abhängig von nationalem Recht, ebenfalls möglich. Diese Begriffe sind für die Zwecke der Beschreibung der Erfindung austauschbar.

Im folgenden werden die Verwendungen des erfindungsgemäßen Kits und Klebers genauer erläutert.

Eine Erkenntnis der vorliegenden Erfindung ist, dass mittels Brückenmolekülen unter Einsatz von (lignolytischen) Polyphenoloxidasen, wie Laccase, unter Ausbildung kovalenter Bindungen mit Polymeren, die mindestens eine freie Aminogruppe aufweisen, eine Vernetzung herbeigeführt werden kann. Daher richtet sich die vorliegende Erfindung zum einem auf einen Kit bzw. Kleber wie oben definiert zur Verwendung für das Verschließen von Wunden.

Der Kit und/oder der Kleber kann zum Kleben von Hart- oder Weichgewebe verwendet werden. Zum Beispiel kann Bindegewebe, Haut, Sehne, Organ, Blutgefäß und/oder Nerv (Weichgewebe) und/oder Zahn und/oder Knochen (Hartgewebe) geklebt werden. Selbstverständlich können auch verschiedene Gewebe miteinander verklebt werden, z.B. Sehnen und Knochen. Eine besonders bevorzugte Anwendung findet sich beim Verkleben von Wundrändern oder beim Kleben von Rupturen verschiedener Organe, wie Leber, Niere oder Milz. Auch die Versorgung von chirurgisch gesetzten Wunden, z.B. nach der Entfernung von Tumoren, ist mit dem erfindungsgemäßen Kit und/oder Kleber möglich. Weitere Einsatzgebiete sind
- in der plastischen, rekonstruktiven und/oder kosmetischen Chirurgie, insbesondere zur Verhinderung der Narbenbildung, die beim Nähen von Wunden induziert wird;
- Verschluss und Abdichtung von Flüssigkeit- und Luftleckagen z. B. Abdichtung von Stichkanalblutungen in der Gefäßchirurgie, bei arteriellen Bypassoperationen oder zur Abdichtung von Lungenleckagen in der Thoraxchirurgie.

Eine weitere vorteilhafte Anwendung des Kits und/oder Klebers ist die flexible, über einen gewissen Zeitraum resorbierbare Abdichtung von Anastomosen und Patchen an Gefäßen bzw. auf Hohlorganen.

Der Kit und/oder Kleber ist auch für die Fixierung von Drug-Delivery Devices und von porösen Trägerstrukturen bzw. Membranen für den potentiellen Einsatz in der regenerativen Medizin (Tissue Engineering) geeignet. Hier ist von besonderem Vorteil, dass eine Verklebung auch mit Oberflächen nicht biogenen Ursprungs erfolgen kann, sofern sie freie Aminogruppen tragen. Freie Aminogruppen können auf solchen Oberflächen nicht biogenen Ursprungs nach im Stand der Technik bekannten Verfahren, z.B. Plasmaverfahren, erhalten werden (Schröder et al., Improved low-pressure microwave plasma assisted amino functionalization of polymers, Plasma processes and polymers, Weinheim, Deutschland, Weinheim: Wiley-VCH, 2005; Schröder et al., Plasma-Induced Surface Functionalization of Polymeric Biomaterials in Ammonia Plasma, Contrib. Plasma Phys. 41, 2001, 562-572; Meyer-Plath et al., Current trends in biomaterial surface functionalization - nitrogen-containing plasma assisted processes with enhanced selectivity, Vacuum 71, 2003, 391-406).

Die Oberflächen z.B. von Implantaten oder von Drug-Delivery Devices, die mit dem Kit und/oder Kleber mit umgebenden Gewebe verbunden werden sollen, sollten einen Gehalt von mindestens 2% freien Aminogruppen aufweisen. Der Begriff Oberfläche beschreibt dabei nicht nur flache Flächen, sondern Grenzschichten eines festen Substrats in jeder Form. Substrate können also z.B. auch Kügelchen (beads) oder komplex geformte Oberflächen sein.

Insbesondere betrifft die Erfindung einen Kit und/oder Kleber wie oben definiert zur Verwendung beim Verkleben von Weich- und Hartgewebe. Bei dem Verkleben von Weich- und Hartgewebe kann es sich unter anderem um das Verkleben von Knochen an Knochen handeln. Dabei werden insbesondere kleine knöcherne Fragmente, die wegen ihrer Größe nicht oder nur unzureichend mit Schrauben fixiert werden können, mit dem erfindungsgemäßen Kit und/oder Kleber fixiert. Im Vergleich zu den metallischen Implantaten ist bei dem Verkleben mittels des erfindungsgemäßen Kit und/oder Klebers keine zweite Operation nach Ausheilung der Fraktur erforderlich. Des Weiteren können mit dem erfindungsgemäßen Kit und/oder Kleber Knochen an Weichgewebe fixiert werden. Insbesondere Knorpeltransplantate (z.B. aus Gewebekulturen), z.B. zur Behandlung von Arthrose, können mittels des erfindungsgemäßen Kit und/oder Klebers bis zum Anwachsen an der Gelenkfläche ausreichend fixiert werden. Besonders vorteilhaft ist in diesem Zusammenhang, dass der Kit und/oder Kleber allmählich resorbiert wird. Darüber hinaus lassen sich mittels der erfindungsgemäßen Kit und/oder Kleber auch Knochen an Implantate fixieren. Speziell bei osteoporotischem Knochen ist die Haltekraft von Schrauben sehr herabgesetzt. Durch eine grossflächige Verklebung der Knochenaußenseite mit dem Implantat (z.B. Platte) mittels des erfindungsgemäßen Kits und/oder Klebers kann die Haltekraft der Schrauben unterstützt werden oder auf Schrauben sogar ganz verzichtet werden. Zellvitalitätsuntersuchungen ergaben eine gute Biokompatibilität des Vernetzungsproduktes.

Demnach umfasst die Erfindung insbesondere auch die Verwendung des erfindungsgemäßen Kits mit den drei Einzelkomponenten "Polymere mit mindestens einer freien Aminogruppe", "Brückenmoleküle" und "Polyphenoloxidasen", wie oben definiert, zur Herstellung des erfindungsgemäßen Klebers für das Verschließen von Wunden. Unter Wunden werden Kontinuitätsunterbrechungen von Körperober-oder -innenflächen, wie z.B. bei Schnittwunden und Brüchen, bezeichnet. Folglich umfasst die Erfindung insbesondere auch die Verwendung des erfindungsgemäßen Kit zur Herstellung des Klebers für das Verkleben von Weich- und Hartgewebe sowie das Verkleben von Knochen mit Implantaten (z.B. Platte), wie oben beschrieben.

Durch die Vernetzung mittels der erfindungsgemäßen Kombination, insbesondere die Verknüpfung von Peptidsequenzen, die die für eine Klebewirkung relevanten Aminosäuren enthalten, mit Hilfe der (lignolytischen) Polyphenoloxidase, wie Laccase, entsteht bei verschiedenen Einsatzgebieten eine sehr feste Verklebung, die aber im Verlauf des Heilungsprozesses resorbiert werden kann. Die anfängliche Härtungsphase, verbunden mit hohen Klebeigenschaften, und die zeitabhängig langsame Resorption im Körper bei fortgeschrittenen Heilungsprozessen sind von besonderem Vorteil.

Der Kleber bzw. der Kit kann bei Knochenbrüchen, zur Fixierung von Gefäßprothesen, bioabbaubaren Implantaten, Kathetern, Stents und anderer Materialien genutzt werden.

Demnach umfasst die Erfindung auch die Verwendung des erfindungsgemäßen Kits mit den drei Einzelkomponenten "Polymere mit mindestens einer freien Aminogruppe", "Brückenmoleküle" und "Polyphenoloxidasen", wie oben definiert, zur Herstellung eines Klebers für Verkleben von Knochenbrüchen und / oder zur Fixierung von Gefäßprothesen, bioabbaubaren Implantaten, Kathetern, Stents und anderer Materialien.

Für das Verkleben von Knochen sowie für die schnelle Integration von Implantaten insbesondere im Bereich der Knochen ist die Akzeptanz des implantierten Materials durch die Knochenzellen von fundamentaler Bedeutung. Dies trifft natürlich auch auf die verwendeten Kleber zu. Durch die schnelle Besiedlung mit Osteoblasten und deren anschließende Ausreifung wird die Knochenneubildung gefördert und der Heilungsprozess verkürzt. Implantate werden daher mit mikrostrukturierten Oberflächen gefertigt. Der erfindungsgemäße Kit oder Kleber erhält diese Struktur und unterstützt die Knochenneubildung. Für die Produktion von Kollagen, das für die Knochenheilung unentbehrlich ist, sind die Aminosäuren Lysin und Prolin notwendig.

Bevorzugt wird deshalb für dieses Einsatzgebiet (Lysin-Tyrosin)ₙ als Polymer verwendet. Bei Verwendung von (Lysin-Tyrosin)ₙ als Polymer ist Lysin an der Klebestelle in hoher Konzentration vorhanden. Daraus resultiert eine positive Beeinflussung der Knochenbildung. Die gebildete extrazelluläre Matrix wird über Integrine, wie z.B. α1β1 oder α3β1, anhand der RDG-Sequenz (Arg-Gly-Asp) erkannt. Dadurch wird eine Signalkaskade in Gang gesetzt, die in der Zelle Veränderungen am Zytoskelett auslöst und zwischen Proliferationsstadium und Differenzierungsstadium umschaltet. Die gebildete Proteinmatrix dient außerdem der Einlagerung von Calciumsalzen.

Das ermöglicht die Anwendung bei komplizierten Brüchen und die Einfügung von Knochensplittern. Gerade bei der Operation komplizierter Brüche sind die Vorteile der erfindungsgemäßen Kombination von Blutstillung und hoher Klebekraft von großer Bedeutung.

In einem weiteren Aspekt richtet sich die Erfindung auf einen wie oben definierten Kit oder Kleber, welcher neben den oben definierten Einzelkomponenten a), b) und c) als weitere Einzelkomponente d) ein neuartiges Zeolithgranulat, das sich zur Blutstillung eignet, umfasst.

Durch dieses neuartige Zeolithgranulat wird insbesondere eine rasche Verklebung von Wundrändern erreicht.

Bei dem neuartigen Zeolithgranulat handelt es sich um ein Zeolithgranulat, das mindestens 70 Gew.-% an Zeolith, insbesondere mit Anteilen von Klinoptilolith, Chabasit und Mordenit, und einen Mikroporenraum mit einer mittleren Porengröße von 0,3 bis 0,5 nm aufweist. Das Zeolithgranulat wird durch die Schritte Vermahlen, Fraktionierung und Dehydratisierung bei Temperaturen unter 200 °C erhalten. Vorzugsweise ist dieses Zeolithgranulat hydratfrei, wobei dieser Zustand vorzugsweise nach Trocknung und Vorzerkleinerung durch eine schonende Dehydration bei Temperaturen unter 200 °C erreicht wird. Bei der schonenden Dehydration werden insbesondere verschiedene Temperaturintervalle unter 200 °C angesetzt. Besonders bevorzugt werden Zeolithgranulate eingesetzt, die aus der Fraktion mit Kornband (mittlerer Korngröße) 0,8 - 0,2 mm stammen.

Bezüglich Reinheit dieser Zeolithgranulate sind die Anforderungen nach US Pharmacop. bzw. Europ. Pharmacop. zu erfüllen.

Es hat sich herausgestellt, dass im Mikroporenraum dieser Zeolithgranulate bestimmte an der Blutgerinnung beteiligte Faktoren selektiv angereichert werden. Das führt zu einer Aktivierung der physiologischen Blutgerinnung, die zu einer den herkömmlichen Hämostyptika deutlich überlegenen Blutstillung führt. Von besonderem Vorteil ist dabei, dass keinerlei toxische Stoffe an die Wunde abgegeben werden. Bei der beschriebenen Aufbereitung wird ein Zeolithgranulat mit starken elektrostatischen Feldern im Kristallgitter erhalten, das selbst schwere Blutungen innerhalb 1 min stoppen kann. Überraschenderweise hat sich herausgestellt, dass die in der Refindung verwendeten Zeolithgranulate den am Markt befindlichen Produkten auf Basis mikroporosierter synthetischer Lithiumsilikate (EP 1176991 A1, WO 00/69480, US 6833486 B1), z.B. CERDAC, durch eine 5 bis 10-fach höhere Kapillarkraftwirkung überlegen sind. In Folge der partiellen Dehydratisierung tritt keine exotherme Reaktion auf, die Gewebeschäden durch Verbrennung verursachen kann.

Durch die beschriebene erfindungsgemäße Aufbereitung können nunmehr Naturzeolithe erstmals auf kostengünstigen direkten Weg als blutstillende Mittel und Mittel zur Wundversorgung bereitgestellt werden.

Wie oben bereits erwähnt hat sich herausgestellt, dass durch die in der Erfindung verwendeten Zeolithgranulate nicht nur eine Blutstillung, sondern auch eine rasche Verklebung der Wundränder erreicht wird. Nach der dem Verkleben der Wundränder kann der Mineralstoff in beispielsweise durch Ablösung der schorfähnlichen Wundabdeckung und Absaugung der Reste vollständig entfernt werden. Es ist bei dieser Ausführungsform der Erfindung möglich, die Zeolithe in einer Umhüllung z.B. auf Zellulosebasis oder auf textiler Basis oder auf Basis eines Collagenvlieses anzuwenden. Damit ist die anschließende vollständige Entfernung besonders problemlos möglich.

Durch die Aktivierung des Fibrinsystems sind die Wundränder miteinander verklebt. Die Reißfestigkeit des Wundverschlusses ist aber bei Verwendung eines Zeolithgranulats alleine geringer als bei herkömmlichen Fibrinklebern.

Um die Reißfestigkeit zu erhöhen, ist es zweckmäßig, die Zeolithgranulate in dem oben beschriebenen Kit oder Kleber einzusetzen. Durch diese Kombination lassen sich besonders wirkungsvolle hämostatische Wundkleber zur Wundbehandlung, Wundversiegelung und Blutstillung bereitstellen.
Demnach umfasst die Erfindung auch den erfindungsgemäßen Kit / Kleber mit Zeolithgranulat zur Verwendung zum Verkleben von Wunden sowie zum Abdichten von Anastomosen und Patchen an Gefäßen bzw. auf Hohlorganen.

Insofern richtet sich die vorliegende Erfindung in einer weiteren Ausführungsform auf ein Kit, welches die folgenden Einzelkomponenten umfasst:
a) Polymere mit mindestens einer freien Aminogruppe,
b) Brückenmoleküle ausgewählt aus der Gruppe bestehend aus monocyclischen ortho-Dihydroxyaromaten, monocyclischen para-Dihydroxyaromaten, bicyclischen Monohydroxyaromaten, polycyclischen Monohydroxyaromaten, bicyclischen Dihydroxyaromaten, polycyclischen Dihydroxyaromaten, bicyclischen Trihydroxyaromaten, polycyclischen Trihydroxyaromaten, und deren Mischungen,
c) Polyphenoloxidasen, insbesondere lignolytische Polyphenoloxidasen, und
d) Zeolithgranulat wie oben beschrieben
wobei die Einzelkomponenten b), c) und d), d.h. die Brückenmoleküle mit den lignolytische Polyphenoloxidasen und das Zeolithgranulat, nicht miteinander in Kontakt stehen.

Bezüglich der einzelnen Komponenten und ihren bevorzugten Ausführungsformen wird auf die obigen Ausführungen verwiesen.

Dabei ist bevorzugt, dass das Zeolithgranulat (z.B. Naturzeolith-Feingranulate) nicht in Kontakt mit den anderen Einzelkomponenten des Kits steht, d.h. es ist bevorzugt, dass die Zeolithe gesondert abgepackt und damit gesondert verwendet werden. Dabei sorgen Naturzeolith-Feingranulate auf Grund ihrer hohen Kapillarkraft durch Konzentration von Gerinnungsfaktoren für einen raschen Wundverschluss. Danach können die Zeolithe problemlos wieder entfernt werden. Es wird ein blutfreies übersichtliches Operationsfeld erhalten. Vorteilhaft wirkt sich weiterhin aus, dass eine Korrekturmöglichkeit z.B. vor der Zugabe der unvernetzten Polymere - falls gewünscht - noch gegeben ist.

Werden in diese zunächst provisorisch verschlossene Wunde die Einzelkomponenten a) bis c) des Kits gegeben, kommt es unter Ausbildung kovalenter Bindungen zu einem dauerhaften, belastbaren, andererseits aber auch resorbierbaren Wundverschluss.

Natürlich sind auch anderen Mittel zur Blutstillung als die hier beschriebenen Zeolithgranulate denkbar, die es ermöglichen den Blutaustritt stark zu senken und dadurch einen Abtransport der (lignolytischen) Polyphenoloxidasen, insbesondere Laccase, am Einsatzort zu verhindern. Jedoch zeigt das beschriebene Vorgehen - erst Blutstillung und Wundverschluss mit Naturzeolithfeingranulaten und anschließend dauerhafte Verklebung unter Einwirkung der Polyphenoloxidase, wie Laccase - viele Vorteile und ist konkurrierenden Verfahren auch unter Kostengesichtpunkten überlegen. Der erfindungsgemäße hämostatische Kit zur Wundbehandlung, Wundversiegelung sowie zum Einkleben von Hart- und Weichteilgewebe und von Implantaten ist darüber hinaus weitaus leichter handhabbar. Vorteilhaft einsetzbar sind die hämostatischen Kleber
- in der plastischen, rekonstruktiven und/oder kosmetischen Chirurgie, insbesondere zur Verhinderung der Narbenbildung, die beim Nähen von Wunden induziert wird
- bei Verschluss und Abdichtung von Flüssigkeit- und Luftleckagen z. B. Abdichtung von Stichkanalblutungen in der Gefäßchirurgie, bei arteriellen Bypassoperationen oder zur Abdichtung von Lungenleckagen in der Thoraxchirurgie.

Der erfindungsgemäße Kit ist eine Alternative sowohl zur Elektrokoagulation als auch zum Wundverschluss mit Nadel und Faden.

Folglich umfasst die vorliegende Erfindung auch die Verwendung des erfindungsgemäßen Kits, welcher die Einzelkomponenten a) bis d) umfasst, zur Herstellung eines Klebers für das Verschließen von Wunden. Des Weiteren umfasst die Erfindung insbesondere auch die Verwendung des erfindungsgemäßen Kit, welcher die Einzelkomponenten a) bis d) umfasst, zur Herstellung des Klebers für das Verkleben von Weich- und Hartgewebe sowie das Verkleben von Knochen mit Implantaten (z.B. Platte), wie oben beschrieben.

Darüber hinaus richtet sich die vorliegende Erfindung auch auf einen Kleber der die Komponenten a) bis d), wie oben beschrieben, umfasst, wobei mindestens die Komponenten b) bis c) bzw. a) bis c), vorzugsweise alle Einzelkomponenten a) bis d), miteinander in Kontakt stehen.

Die hohe Kapillarkraft der Naturzeolith-Feingranulate ist auch für den Einsatz zur Wundversorgung, insbesondere zur Versorgung von schwer heilenden und bereits chronifizierten Wunden von besonderer Bedeutung. Besonders angezeigt ist der Einsatz der der Naturzeolith-Feingranulate bei Wunden mit einer Exsudation, d.h. einem Austritt von Blutbestandteilen an einer entzündeten Wunde. Oft ist eine Exsudation ein Anzeichen für eine Infektion an einer Verletzung. Die wie hier beschrieben erhaltene Mikroporenstruktur der Zeolithgranulate ermöglicht die Aufnahme von Wundexsudat, das bei "nässenden" Wunden im Überschuss abgegeben wird. Gleichzeitig sorgt die Aufnahme des überschüssigen Exsudats für eine feuchte Atmosphäre über der Wunde, was für die Wundheilung von Bedeutung ist.

Weitere erfindungsgemäße Verwendungen sind:
- als Mittel zur Ersten Hilfe nach Unfällen
- zur Wundsanierung bei infizierten Wunden.

Völlig überraschend wurde gefunden, dass Zeolithe mit der Porenweite 0,3 bis 0,5 nm insbesondere im Verhältnis 1 : 10 bis 10 : 1 mit der Biomasse aus aquatischen Organismen, wie weiter unten definiert, gemischt werden können, ohne an blutstillender Wirkung oder Kapillarkraft zu verlieren.

Demnach kann der Einsatz der neuartigen Zeolithgranulate erweitert werden, indem man diese Materialien mit Biomasse substituiert. Folglich umfasst die vorliegende Erfindung auch Zeolithgranulate, die zusätzlich Biomasse, insbesondere im Verhältnis Zeolith zu Biomasse von 1 : 10 bis 10 : 1, enthalten. Erfindungsgemäß werden bevorzugt Mikroalgen mit antibakterieller Aktivität ausgewählt, deren Biomassen lyophilisiert und gemahlen und/oder gemäß der technischen Lehre von Lukowski et al.: Pharmaceutically or cosmetically active agents obtained from lipidcontaining marine organisms, (US 10/507,061) aufbereitet wird.

Durch Mischen der Naturzeolith-Feingranulate mit derartig aufbereiteten Biomassen, die antimikrobielle Wirkstoffe enthalten, gelingt es, eine Kombination bereitzustellen, die die Aufnahme des überschüssigen Wundexsudats gewährleistet, die Wunde feucht hält, die Keimbesiedlung der Wunde verhindert, entzündungshemmend und immunstimulierend wirkt und die Zellproliferation fördert. Vorzugsweise werden bei der Aufbereitung gemäß US 10/507,061 Mikroalgen mit einem Lipidanteil > 20 % und vielen ungesättigten Fettsäuren, und/oder mit einem hohen Anteil an Poly-N-Acetyl-Glucosamine ausgewählt. Damit kann die antibaktakterielle Aktivität der Fettsäuren vorteilhaft für diese Aufgabenstellung genutzt werden. Polysaccharide, vorteilhafterweise aus Poly-N-Acetyl-Glucosaminen oder Chitosan haben eine große innere Oberfläche. Aufgrund der Absorption an diese große Fläche kommt es zu einer synergistischen Wirkung bei der Konzentration von Gerinnungsfaktoren ebenso wie bei der Aufnahme von Wundsekret sowie von bakteriellen Toxinen. Darüber hinaus haben diese Polysacharide eine immunstimulierende Wirkung. Gemäß einer bevorzugten Ausführungsform der Erfindung werden gefriergetrocknete Biomassen der Grünalge Chlamydomonas und / oder der Mikroalgen Spirulina und / oder Anabaena eingesetzt. Bei der Anwendung der Zeolithe mit oder ohne Biomasse auf einer blutenden Wunde entsteht durch die Aktivierung des Fibrinsystems eine feste schorfähnliche Abdeckung, die die Wunde zuverlässig vor einer bakteriellen Infektion schützt. Diese Abdeckung wird im Laufe der Wundheilung abgestoßen.

In einer geeigneten Anwendungsform können die Feingranulate aus Naturzeolithen und Mikroalgenbiomassen auch eingesetzt werden, um stark infektiöse, nässende Wunden zu therapieren.

Bei verschiedenen Einsatzgebieten kann der Kit und/oder Kleber diese Kombination von Zeolithgranulat und Biomasse in allen im Detail oben beschriebenen Ausführungsformen enthalten.

Die Merkmale der Erfindung gehen außer aus den Ansprüchen auch aus der Beschreibung hervor, wobei die einzelnen Merkmale jeweils für sich allein oder zu mehreren in Form von Kombinationen vorteilhafte schutzfähige Ausführungen darstellen, für die mit dieser Schrift Schutz beantragt wird.

Die Erfindung soll anhand von Ausführungsbeispielen erläutert werden, ohne auf diese Beispiele beschränkt zu sein.

### Beispiele

### Beispiel 1: Herstellung der Naturzeolithfeingranulate

Selektiv abgebaute Mineralien mit ≥ 70 % Zeolithanteilen, vorzugsweise Klinoptilolith, Chabasit und Mordenit werden nach Trocknung und Vorzerkleinerung durch Fraktionierung mit Kornband 0,8 - 0,2 mm gewonnen. Bezüglich Reinheit sind die Anforderungen nach US Pharmacop. bzw. Europ. Pharmacop. zu erfüllen. Durch schonende thermische Behandlung in ausgewählten Temperaturintervallen bei Temperaturen < 200 °C erfolgt eine Teildehydratisierung unter Erhalt der Kristallgitterstruktur.

### Beispiel 2: Prüfung des Mikroporenvolumens und der Korngrößenverteilung

Die nach Beispiel 1 hergestellten Produkte verfügen über 25 - 50% Mikroporenraum (bezogen auf das Gesamtvolumen) und durchschnittliche Nanoporendurchmesser im Bereich 0,3 - 0,5 nm.

Nanoporengröße und Feingranulataufbau ermöglichen eine maximale Blutadsorption und schnelle Durchdringung des Adsorberfeingranulates.

Die Kapillarkraftwirkung ist 5 bis 10fach höher als bei dem vergleichsweise untersuchten Handelspräparat CERDAC.

### Beispiel 3: Prüfung der Feingranulate auf Eignung zur Blutstillung

### Methodik:

Es wurden 2 Feingranulate, hergestellt nach Beispiel 1, geprüft.

Die Untersuchungen erfolgten an der Rattenleber als einem Prototyp für ein stark durchblutetes Organ. Zu diesem Zweck wurden Ratten narkotisiert. Nach Öffnung des Bauchraums wurde mit dem Skalpell ein etwa 1 cm langer Schnitt gemacht. Die Blutung wurde gefilmt. Die Zeit bis zur Blutstillung wurde gemessen.

### Ergebnis:

Bei Anwendung der Granulate wird eine Blutstillung innerhalb von 1 min erreicht (Kontrolle ohne Behandlung bis zu 8 min). Es bildet sich eine schorfähnliche Verkrustung, die die Wunde schützt.

### Beispiel 4: In vitro-Prüfung der Feingranulate auf Eignung zur Blutstillung

Bei Untersuchungen an verschiedenen Blutplasmen wurden folgende Ergebnisse erzielt:
a) Normalplasma (mit Vorinkubation von 2 min)

| | Normalplasma | Granulat 1 | Granulat 2 |
|---|---|---|---|
| Rekalzifizierungszeit | 115 s | 60 s | 60 s |
| PTT nach 3 min Inkubation | - | 15 s | sofort clot |

| | | | |
|---|---|---|---|
| Clot= Gerinnung | | | |

b) Mangelplasma

| | Granulat 1 | Granulat 2 |
|---|---|---|
| F - VII MPI | Clot nach 5 min | Clot nach 210 s |

| | | |
|---|---|---|
| Ohne Granulat erfolgte keine Gerinnung. | | |

c)Heparinplasma (mit 2 und 3 min Vorinkubation)

| | ohne Granulat | Granulat 1 | | Granulat 2 | |
|---|---|---|---|---|---|
| | | 2 min | 3 min | 2 min | 3 min |
| 1. | 68 s | 46 s | 43 s | 65 s | 28 s |
| 2. | 45 s | 38 s | 17s | 37 s | 10 s |

| | | | | | |
|---|---|---|---|---|---|
| Die Ergebnisse belegen, dass das endogene Gerinnungssystem aktiviert wird, wobei der Mangelfaktor F - VII und Gewebsfaktor zu verstärkter Trombin- und Fibrinbildung führen. Es erfolgt eine relative Fibrinkonzentrationserhöhung durch die mineralische Wundauflage nach Beispiel 1. | | | | | |

### Beispiel 5: Prüfung von Extrakten aus Mikroalgen auf antibakterielle Wirksamkeit

### Methodik:

Verschiedene Staphylokokken-Stämme wurden mittels Whitley Automatic Spiral-Plater im linearen Modus gleichmäßig auf der Agarplatte (Müller-Hinton-II-Agar Fertigplatten von Becton Dickinson) verteilt.

Acetatgewebe, wie es für die Herstellung von Wundauflagen verwendet wird, wird mit 2 mg Extrakt entsprechenden Menge Extraktlösung betropft und trocknen gelassen.

Zur Herstellung der Extrakte wurden gefriergetrocknete Biomassen der Grünalge Chlamydomonas, der Mikroalgen Spirulina (Blue Biotech Büsum) und Anabaena (Stammsammlung des Instituts für Pharmazie der Ernst-Moritz-Arndt-Universität Greifswald) verwendet. Zur Extraktion mit n-Hexan wurde der Extraktor DIONEX ASE 200 genutzt. Die Extraktlösung wurde mittels Rotationsverdampfer vom Lösungsmittel befreit.

### Ergebnis:

Bei der Kontrolle werden 70- 120 Keime/cm² gezählt. Unter den beschichteten Wundauflagen lassen sich keine als Erreger von Wundinfektionen gefürchteten Methicillin-resistenten Staphylokkus-aureus-Stämme (MRSA) nachweisen. Bei anderen Staphylokokken war das Keimwachstum auf 10 bis 20 % der Ausgangskeimzahl reduziert.

### Beispiel 6: Herstellung von Feingranulaten aus Naturzeolithen und der Biomasse von Mikroalgen

Den nach Beispiel 1 hergestellten Mineralfeingranulaten können Mikroalgenbiomassen mit einem Lipidanteil > 20 % im Verhältnis 1:10 bis 10:1 zugemischt werden. Hierdurch wird die Keimbesiedlung der Wunden verhindert.

### Beispiel 7: Prüfung der Feingranulate auf Eignung zur Blutstillung und zum Wundverschluss

### a) Methodik:

Es wurden 2 Feingranulate, hergestellt nach Beispiel 1, geprüft.

Die Untersuchungen erfolgten an der Rattenleber als einem Prototyp für ein stark durchblutetes Organ. Zu diesem Zweck wurden Ratten narkotisiert. Nach Öffnung des Bauchraums wurde mit dem Skalpell ein etwa 1 cm langer Schnitt gemacht. Die Blutung wurde gefilmt. Die Zeit bis zur Blutstillung wurde gemessen. Der Wundverschluss wurde visuell beurteilt.

### Ergebnis:

Bei Anwendung der Granulate wird eine Blutstillung innerhalb von 1 min erreicht (Kontrolle bis zu 8 min). Es bildet sich eine schorfähnliche Verkrustung, die die Wunde schützt.
b) In weiteren Versuchen, wurde die Verkrustung nach 1 min mit dem Skalpell entfernt.

### Ergebnis:

Es wird eine nicht mehr blutende, oberflächlich geschlossene Wunde mit visuell gutem Wundverschluss erhalten. Der Wundverschluss ist bei Feingranulaten auf Mineralstoff/Algenbiomasse-Basis besser als bei der Verwendung der ausschließlich auf mineralischer Basis hergestellter Präparate.
c) In weiteren Versuchen wurden die Feingranulate durch ein Zellulosetuch von der Wunde getrennt.

### Ergebnis:

Auch unter diesen Bedingungen wird eine Blutstillung in weniger als einer Minute erreicht. Es entsteht ein sehr übersichtliches Operationsgebiet, so dass eine feste Verklebung der Wunde mit dem erfindungsgemäßen Gewebekleber problemlos erfolgen kann.

### Beispiel 8. Herstellung der Kleberpräpolymere und Durchführung der Klebereaktion für die Weichgewebe- und Hartgewebeklebung

Der entwickelte Kleber resultiert aus einer Mischung der einzelnen Komponenten Oligopeptid **1** (Aminosäuresequenz aus sich wiederholenden Dipeptideinheiten von Tyrosin und Lysin mit n = 5 oder n = 10), Brückenmolekül **2** (2,5-Dihydroxy-N-(2-hydroxyethyl)-benzamid) und dem Enzym Laccase (Schema 5). Die Untersuchungen wurden in Phosphatpuffer durchgeführt.

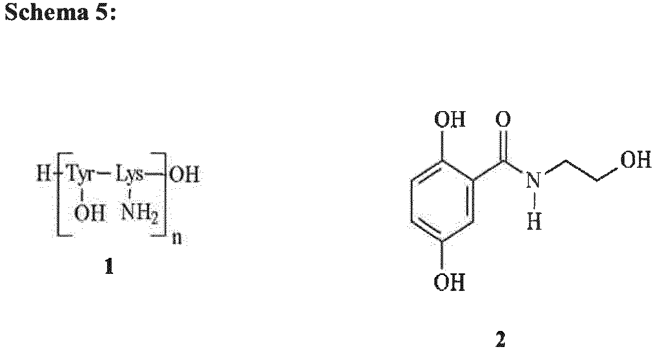

Die Klebewirkung erfolgt durch die enzymatische Reaktion der Laccase mit dem Brückenmolekül, das mit dem Oligopeptid verknüpft wird.

Es wurden folgende Mischungsverhältnisse eingesetzt:
[Tyr-Lys]ₙ, n=4-35, 8,5 mM; 2,5-Dihydroxy-N-(2-hydroxyethyl)-benzamid 12,5 mM; Enzym: 0,32 U (156 nmol ml⁻¹ min⁻¹). Der Lösungsmittelanteil an Methanol betrug 10% (v/v).

### Beispiel 9: Prüfung der Festigkeit der Klebefuge

Zur Prüfung der Festigkeit der Klebefuge im Zugversuch mussten die zu untersuchenden Gewebeteile vorher fixiert werden. Porcines Weichgewebe wurde mit Cyanacrylatkleber an Probenhaltern aus Polymethylmethacrylat (PMMA) fixiert bzw. Knochenplatten aus Knochen bovinen Ursprungs wurden in mechanischen Klemmen eingespannt. Anschließend wurde die zu untersuchende Klebermischung (s. Bsp. 8) auf das Gewebe aufgetragen. Bei der Weichgewebeklebung wurde nach 10 Minuten die Festigkeit der Klebefuge unter Zugbeanspruchung an der Prüfmaschine Zwick BZ2.5/TN1S (Prüfgeschwindigkeit: 10 mm/min) ermittelt. Die mechanische Testung der Klebefuge nach Hartgewebeklebung erfolgte bei Zug-Scherbeanspruchung mit einer Prüfgeschwindigkeit von 5 mm/min.

Ergebnis: Es wird ein doppelt so starke Festigkeit erreicht wie bei den vergleichend untersuchten kommerziellen Fibrinklebern.

### Beispiel 10: Diffusionshemmtest mit dem erfindungsgemäß dotierten Collagenvlies im Vergleich zu einem mit einem konventionellen Antibiotikum dotiertem Vlies.

### Methodik:

Es wurde der Agardiffusionstest nach Burkhardt (Burkhardt, F. (Hrsg.) Mikrobiologische Diagnostik. Georg-Thieme Verlag Stuttgart, New York 1992, S 724) durchgeführt. Für die Testung wurde Mueller-Hinton II-Agar in Stacker-Petrischalen (Becton Dickinson Microbiology Systems, Cockeysville, USA) eingesetzt. Als Teststamm wurde S. aureus Stamm ATCC 6538 ausgewählt. Die Einsaat dieses Teststamms wurde so gewählt, daß sich nach 1620 h Bebrütung dicht stehende, jedoch nicht konfluierende Einzelkolonien entwickeln. Nach dem Trocknen der beimpften Nährböden werden die dotierten Collagenvliese auf die Agaroberfläche aufgelegt. Nach 18 + 2 h Bebrütung bei 36 °C werden die Hemmhöfe gemessen.

**Ergebnisse:**

| | Hemmhof gegen S. aureus ATCC 6538 | |
|---|---|---|
| Dotierung | Gentamycin | Erfindungsgemäß dotiertes Vlies |
| 15% | | 30 |
| 20% | 24 | 36 |
| 30% | | 38 |
| 40% | 38 | |

Das erfindungsgemäße Vlies weist folgende Vorteile auf:
- verbesserte antimikrobielle Wirksamkeit, auch bei multiresistenten Keimen
- sehr gute hämostyptische Wirkung
- gute Haftung auf der Wunde bei einfacher Applikation.

### Beispiel 11: Prüfung von mit Polyphenolen dotierten Oberflächen auf antibakterielle Wirksamkeit

### Methodik:

Verschiedene Staphylokokken-Stämme wurden mittels Whitley Automatic Spiral-Plater im linearen Modus gleichmäßig auf der Agarplatte (Müller-Hinton-II-Agar Fertigplatten von Becton Dickinson) verteilt.

Polymere Biomaterialien (jeweils 1 cm²) werden nach Contrib. Plasma Phys. 41, 2001, 562-572 im Ammonik-Plasma funktionalisiert. Danach erfolgte die Umsetzung mit 2,5-Dihydroxy-N-(2-hydroxyethyl)-benzamid (12,5 mM) unter dem Einfluss der Laccase (0,32 U (156 nmol ml⁻¹ min⁻¹)). Der Lösungsmittelanteil an Methanol betrug 10% (v/v).

Nach dem Trocknen werden die Platten auf den Agar gelegt.

### Ergebnis:

Bei der Kontrolle werden 120 Keime/cm² gezählt. Unter den beschichteten Oberflächen lassen sich keine Keime nachweisen.

### Beispiel 12: Verwendung der erfindungsgemäßen Kombination zur Versorgung blutender Wunden.

### Methodik:

Ratten werden wie in Beispiel 7 narkotisiert, der Bauchraum geöffnet und ein Schnitt in der Leber gesetzt. Die Blutung wird wie in Beispiel 7 c gestillt. Die sich bildende Kruste wird entfernt. Anschließend wird der Schnitt mit Wundkleber geklebt.

Dafür werden die in Beispiel 8 beschriebenen Oligopeptide in PBS (Phosphate Buffered Saline, 2,7 M NaCl, 54 mM KCl, 87 mM Na₂HPO₄, 30 mM KH₂PO₄, pH 7.4) aufgenommen und Laccase zugegeben (Komponente 1). 2,5-Dihydroxy-N-(2-hydroxyethyl)-benzamid wird in PBS gelöst (Komponente 2). Es werden verschiedene Verhältnisse von Oligopeptid/2,5-Dihydroxy-N-(2-hydroxyethyl)-benzamid/Laccase getestet, z.B. 1/1/0,1; 1/2/0,5; 1/10/1. Die Menge an Lösungsmittel wird minimal gewählt, um eine möglichst konzentrierte Lösung der Komponenten zu erreichen. Die Komponenten werden in eine Zweikomponentenspritze mit Mischextruder vom Typ Mixpac (Mixpac Systems, Rotkreuz, Schweiz) gegeben und in den Wundspalt eingebracht.

Ergebnis: Bereits durch das Hämostyptikum erfolgt ein guter Wundverschluss. Der Kleber kann direkt in den resultierenden engen Spalt gegeben werden. Es resultiert eine sehr feste Verklebung der Wundränder.

### Beispiel 13: Verklebung von Collagen

### Methodik:

Collagenfolie (DOT GmbH) wurde auf einer ebenen Unterlage fixiert.

Die Collagenfolie wurde in 8 Segmente (je 4 in zwei Reihen) unterteilt und der Kleber direkt auf der Folie (unter Ausnutzung der Oberflächenspannung) zusammengemischt. Der Kleber war in Phosphat-Citrat-Puffer gelöst.

Es wurden unterschiedliche Konzentrationen Brückenmoleküls (Laccase-Substrat) getestet. Das Kleberprotein hatte ein MW von ca. 4000D.

Anschließend wurden kleine Collagenfolienstücke aufgebracht, leicht angedrückt und trocknen gelassen.

### Ergebnis:

Es wurde beim Verkleben von Collagen eine feste Bindung erreicht.

Die Verklebung erfolgt auch ohne Zugabe von Kleberproteinen. Durch die Kleberproteine wird jedoch die Bindungskraft verstärkt.

### Beispiel 14: Mischung einer Collagenlösung mit dem Kleber

Mikroskopisch ist nach dem Vermischen der Collagenlösung mit dem Kleber eine starke Strukturierung zu erkennen. Die gelösten und zufällig angeordneten Collagenmoleküle werden parallelisiert - unter Ausbildung von Fasern.

### Beispiel 15: Verkleben von Collagen und Polystyren

### Methodik:

Ein Tropfen (20µl) Citrat-Phosphat-Puffer (Kontrolle) bzw. Kleberlösung (Puffer, Laccase, Laccase-Substrat) wurde auf die Oberfläche einer 60mm TCPS-Zellkulturschale aufgebracht und je ein kleines Stück Collagenfolie (8x4mm) aufgesetzt und leicht angedrückt und dann trocknen gelassen.

### Ergebnis:

Trotz der absolut glatten und inerten Oberfläche wurde eine Verklebung erreicht. Das Collagen wurde dauerhaft mit dem Untergrund verbunden.

### Beispiel 16: Zelladhäsion an den mit Collagen beladenen Oberflächen

### Methodik:

Die Zelladhäsion wurde mit einer Fibroblasten-Zelllinie (FL-Zellen) untersucht.

Die Zelladhäsion erfolgte an 60 mm TCPS-Zellkulturschale, die nach Beispiel 15 mit Collagen beschichtet war. Nach Anfärbung der Zellen mit Kristallviolett erfolgte die Auswertung mit dem CellExplorer.

### Ergebnis:

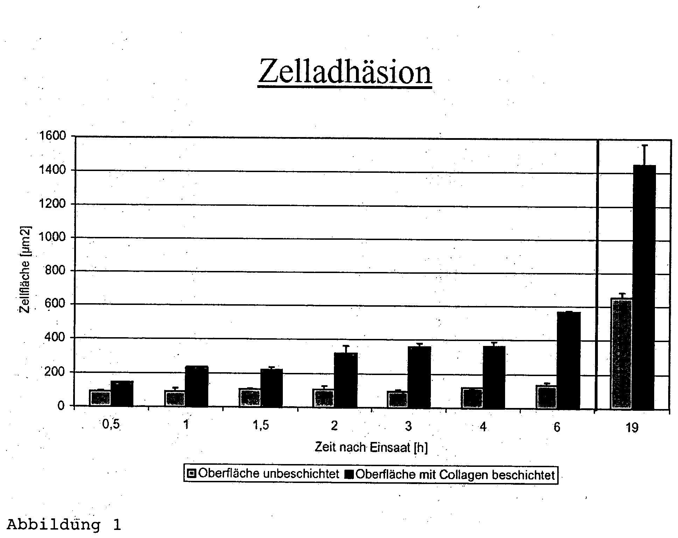

Die FL-Zellen werden stärker gebunden (siehe Abbildung 1).

## Patentansprüche

1. Kit umfassend die Einzelkomponenten
a) Polymere mit mindestens einer freien Aminogruppe,
b) Brückenmoleküle ausgewählt aus der Gruppe bestehend aus monocyclischen ortho-Dihydroxyaromaten, monocyclischen para-Dihydroxyaromaten, bicyclischen Monohydroxyaromaten, polycyclischen Monohydroxyaromaten, bicyclischen Dihydroxyaromaten, polycyclischen Dihydroxyaromaten, bicyclischen Trihydroxyaromaten, polycyclischen Trihydroxyaromaten, und deren Mischungen, und
c) Polyphenoloxidasen,
wobei die Einzelkomponenten b) und c) nicht in Kontakt stehen.

2. Kit nach Anspruch 1, worin die freie Aminogruppe durch eine Diaminosäure erhalten wird.

3. Kit nach einem der Ansprüche 1 bis 2,
worin die Polymere (a) mit den Brückenmolekülen (b) oder mit den Polyphenoloxidasen (c) in Kontakt stehen.

4. Kit nach einem der vorhergehenden Ansprüche 1 bis 3,
worin der Kit eine Spritze darstellt, die zwei Kanülen aufweist, über die die Brückenmoleküle (b) und die Polyphenoloxidasen (c) getrennt abgegeben werden können.

5. Kleber umfassend die Einzelkomponenten
a) Polymere mit mindestens einer freien Aminogruppe, wobei die Polymere nicht Bestandteil des menschlichen Körpers sind,
b) Brückenmoleküle ausgewählt aus der Gruppe bestehend aus monocyclischen ortho-Dihydroxyaromaten, monocyclischen para-Dihydroxyaromaten, bicyclischen Monohydroxyaromaten, polycyclischen Monohydroxyaromaten, bicyclischen Dihydroxyaromaten, polycyclischen Dihydroxyaromaten, bicyclischen Trihydroxyaromaten, polycyclischen Trihydroxyaromaten, und deren Mischungen, und
c) Polyphenoloxidasen,
wobei die Einzelkomponenten b) und c) in Kontakt stehen.

6. Kleber nach Anspruch 5, worin die Einzelkomponenten a) bis c) in Kontakt stehen.

7. Kit oder Kleber nach einem der vorhergehenden Ansprüche, worin der Kit oder Kleber als weitere Einzelkomponente d) ein Zeolithgranulat mit einem Zeolithanteil von mindestens 70 Gew.-% und einer mittleren Porengröße von 0,3 bis 0,5 nm, wobei das Zeolithgranulat durch die Schritte Vermahlen, Fraktionierung und Dehydratisierung bei Temperaturen unter 200 °C erhalten wird, umfasst.

8. Kit oder Kleber nach Anspruch 7, wobei das Zeolithgranulat mindestens zum Teil aus Klinoptilolith, Chabasit und Mordenit, oder Mischungen davon, hergestellt ist.

9. Kit oder Kleber nach Anspruch 7 oder 8, wobei das Zeolithgranulat eine mittlere Korngröße von 0,2 mm bis 0,8 mm hat.

10. Kit oder Kleber nach einem der vorhergehenden Ansprüche 7 bis 9, worin das Zeolithgranulat Biomassen aus aquatischen Organismen umfasst.

11. Kit oder Kleber nach einem der vorhergehenden Ansprüche 7 bis 10, worin sich das Zeolithgranulat in einer Umhüllung, vorzugsweise auf Zellulosebasis oder auf textiler Basis oder einem Collagenvlies, befindet.

12. Kit nach einem der vorhergehenden Ansprüche 7 bis 11, worin das Zeolithgranulat nicht in Kontakt mit den anderen Einzelkomponenten a) bis c) steht.

13. Kleber nach einem der vorhergehenden Ansprüche 7 bis 11, worin das Zeolithgranulat in Kontakt mit den anderen Einzelkomponenten a) bis c) steht.

14. Kit oder Kleber nach einem der vorhergehenden Ansprüche, worin die Brückenmoleküle (b) Dihydroxyaromaten sind.

15. Kit oder Kleber nach Anspruch 14, worin die Dihydroxyaromaten 2,5-Dihydroxybenzamide, vorzugsweise 2,5-Dihydroxy-N-(2-hydroxyethyl)-benzamid sind.

16. Kit oder Kleber nach einem der vorhergehenden Ansprüche, worin die Polyphenoloxidasen (c) lignolytische Polyphenoloxidasen, vorzugsweise Laccasen sind.

17. Kit oder Kleber nach einem der vorhergehenden Ansprüche, worin die Polymere ausgewählt sind aus der Gruppe bestehend aus Oligopeptide, Peptide und Eiweiße.

18. Kit oder Kleber nach Anspruch 17,
worin die Polymere Lysin-haltige Oligopeptide oder Kollagen sind.

19. Kit oder Kleber nach einem der vorhergehenden Ansprüche zur Verwendung als Arzneimittel.

20. Kit oder Kleber nach Anspruch 19 zur Verwendung für das Schließen von Wunden.

21. Kit oder Kleber nach Anspruch 19
(i) zur Verwendung in der Schaffung eines blutfreien Operationsfelds, oder
(ii) zur Verwendung in der Abdichtung von Anastomosen und Patchen an Gefäßen bzw. auf Hohlorganen, oder
(iii) zur Verwendung in der Wundheilung chronischer Wunden, oder
(iv) zur Verwendung in der Wundsanierung bei infizierten Wunden, oder
(v) zur Verwendung beim Verkleben von Organen, Geweben oder Knochen sowie Knochen mit Implantaten.

22. Kit oder Kleber nach Anspruch 19, umfassend Zeolithgranulat wie in irgendeinem der Ansprüche 7 bis 13 definiert, zur Verwendung als Hämostyptika.

## Claims

1. Kit comprising the individual components
a) polymers having at least one free amino group,
b) bridging molecules selected from the group consisting of monocyclic ortho-dihydroxyaromates, monocyclic para-dihydroxyaromates, bicyclic monohydroxyaromates, polycyclic monohydroxyaromates, bicyclic dihydroxyaromates, polycyclic dihydroxyaromates, bicyclic trihydroxyaromates, polycyclic trihydroxyaromates, and mixtures thereof, and
c) polyphenol oxidases,
wherein the individual components b) and c) are not in contact.

2. Kit according to claim 1, wherein the free amino group is obtained by means of a diamino acid.

3. Kit according to either claim 1 or claim 2,
wherein the polymers (a) are in contact with the bridging molecules (b) or with the polyphenol oxidases (c).

4. Kit according to any of the preceding claims 1 to 3,
wherein the kit is a syringe that comprises two cannulas, by means of which the bridging molecules (b) and the polyphenol oxidases (c) can be separately dispensed.

5. Adhesive comprising the individual components
a) polymers having at least one free amino group, wherein the polymers are not constituents of the human body,
b) bridging molecules selected from the group consisting of monocyclic ortho-dihydroxyaromates, monocyclic para-dihydroxyaromates, bicyclic monohydroxyaromates, polycyclic monohydroxyaromates, bicyclic dihydroxyaromates, polycyclic dihydroxyaromates, bicyclic trihydroxyaromates, polycyclic trihydroxyaromates, and mixtures thereof, and
c) polyphenol oxidases,
wherein the individual components b) and c) are in contact.

6. Adhesive according to claim 5, wherein the individual components a) to c) are in contact.

7. Kit or adhesive according to any of the preceding claims, wherein the kit or adhesive comprises, as a further individual component d), a zeolite granulate having a zeolite content of at least 70 wt.% and an average pore size of from 0.3 nm to 0.5 nm, wherein the zeolite granulate is obtained by the steps of grinding, fractionating and dehydrating at temperatures of below 200 °C.

8. Kit or adhesive according to claim 7, wherein the zeolite granulate is produced at least in part from clinoptilolite, chabazite and mordenite, or mixtures thereof.

9. Kit or adhesive according to either claim 7 or claim 8, wherein the zeolite granulate has an average grain size of from 0.2 mm to 0.8 mm.

10. Kit or adhesive according to any of the preceding claims 7 to 9, wherein the zeolite granulate comprises biomasses made up of aquatic organisms.

11. Kit or adhesive according to any of the preceding claims 7 to 10, wherein the zeolite granulate is in a jacket, which is preferably cellulose-based or textile-based, or in a collagen fleece.

12. Kit according to any of the preceding claims 7 to 11, wherein the zeolite granulate is not in contact with the other individual components a) to c).

13. Adhesive according to any of the preceding claims 7 to 11, wherein the zeolite granulate is in contact with the other individual components a) to c).

14. Kit or adhesive according to any of the preceding claims, wherein the bridging molecules (b) are dihydroxyaromates.

15. Kit or adhesive according to claim 14, wherein the dihydroxyaromates are 2,5-dihydroxybenzamides, preferably 2,5-dihydroxy-N-(2-hydroxyethyl)-benzamide.

16. Kit or adhesive according to any of the preceding claims, wherein the polyphenol oxidases (c) are lignolytic polyphenol oxidases, preferably laccases.

17. Kit or adhesive according to any of the preceding claims, wherein the polymers are selected from the group consisting of oligopeptides, peptides and proteins.

18. Kit or adhesive according to claim 17,
wherein the polymers are lysine-containing oligopeptides or collagen.

19. Kit or adhesive according to any of the preceding claims for being used as a medicament.

20. Kit or adhesive according to claim 19 for being used to close wounds.

21. Kit or adhesive according to claim 19
(i) for being used to provide a blood-free site of operation, or
(ii) for being used to seal anastomoses and patches on vessels and/or on hollow organs, or
(iii) for being used to heal chronic wounds, or
(iv) for being used to clean infected wounds, or
(v) for being used to adhere organs, tissues or bones and to adhere implants to bones.

22. Kit or adhesive according to claim 19, comprising zeolite granulate as defined in any of claims 7 to 13, for being used as haemostatic agents.

## Revendications

1. Kit comprenant les composants individuels :
a) de polymères avec au moins un groupe amino libre,
b) de molécules de pontage choisies dans le groupe constitué des ortho-dihydroxy aromatiques monocycliques, des para-dihydroxy aromatiques monocycliques, des monohydroxy aromatiques bicycliques, des monohydroxy aromatiques polycycliques, des dihydroxy aromatiques bicycliques, des dihydroxy aromatiques polycycliques, des trihydroxy aromatiques bicycliques, des trihydroxy aromatiques polycycliques, et de leurs mélanges, et de
c) polyphénoloxydases,
les composants individuels b) et c) n'étant pas en contact.

2. Kit selon la revendication 1, dans lequel le groupe amino libre est obtenu par un acide di-aminé.

3. Kit selon l'une des revendications 1 à 2,
dans lequel les polymères (a) sont en contact avec les molécules de pontages (b) ou avec les polyphénoloxydases (c).

4. Kit selon l'une des revendications précédentes 1 à 3,
dans lequel le kit représente une seringue, qui présente deux canules, par l'intermédiaire desquelles les molécules de pontage (b) et les polyphénoloxydases (c) peuvent être délivrées de manière séparée.

5. Adhésif comprenant les composants individuels :
a) de polymères avec au moins un groupe amino libre, où les polymères ne sont pas des constituants du corps humain,
b) de molécules de pontages choisies dans le groupe constitué des ortho-dihydroxy aromatiques monocycliques, des para-dihydroxy aromatiques monocycliques, des monohydroxy aromatiques bicycliques, des monohydroxy aromatiques polycycliques, des dihydroxy aromatiques bicycliques, des dihydroxy aromatiques polycycliques, des trihydroxy aromatiques bicycliques, des trihydroxy aromatiques polycycliques, et de leurs mélanges, et
c) de polyphénoloxydases,
les composants individuels b) et c) étant en contact.

6. Adhésif selon la revendication 5, dans lequel les composants individuels a) à c) sont en contact.

7. Kit ou adhésif selon l'une des revendications précédentes, dans lequel le kit ou l'adhésif comprend en tant que composant individuel supplémentaire d) un granulat de zéolithe avec une proportion de zéolithe d'au moins 70 % en poids et une taille de pores moyenne de 0,3 à 0,5 nm, le granulat de zéolithe étant obtenu par les étapes de mouture, de fractionnement et de déshydratation à des températures en dessous de 200 C.

8. Kit ou adhésif selon la revendication 7, où lequel le granulat de zéolithe est fabriqué au moins en partie à base de clinoptilolite, de chabazite et de mordénite, ou de mélanges de ceux-ci.

9. Kit ou adhésif selon la revendication 7 ou 8, dans lequel le granulat de zéolithe a une taille de grain moyenne de 0,2 mm à 0,8 mm.

10. Kit ou adhésif selon l'une des revendications précédentes 7 à 9, dans lequel le granulé de zéolithe comprend des biomasses provenant d'organismes aquatiques.

11. Kit ou adhésif selon l'une des revendications précédentes 7 à 10, dans lequel le granulé de zéolithe se trouve dans une enveloppe, de préférence à base de cellulose ou à base d'un textile ou d'un non-tissé en collagène.

12. Kit selon l'une des revendications précédentes 7 à 11, dans lequel le granulé de zéolithe n'est pas en contact avec les autres composants individuels a) à c).

13. Adhésif selon l'une des revendications précédentes 7 à 11, dans lequel le granulat de zéolithe est en contact avec les autres composants individuels a) à c).

14. Kit ou adhésif selon l'une des revendications précédentes, dans lequel les molécules de pontage (b) sont des dihydroxy aromatiques.

15. Kit ou adhésif selon la revendication 14, dans lequel dihydroxy aromatiques sont des 2,5-dihydroxy benzamide, de préférence le 2,5-dihydroxy-N-(2-hydroxyéthyl)-benzamide.

16. Kit ou adhésif selon l'une des revendications précédentes, dans lequel les polyphénoloxydases (c) sont des polyphénoloxydases lignolytiques, de préférence des laccases.

17. Kit ou adhésif selon l'une des revendications précédentes, dans lequel les polymères sont choisis dans le groupe constitué des oligopeptides, des peptides et des albumines.

18. Kit ou adhésif selon la revendication 17, dans lequel les polymères sont des oligopeptides contenant de la lysine ou du collagène.

19. Kit ou adhésif selon l'une des revendications précédentes, pour une utilisation en tant que produit pharmaceutique.

20. Kit ou adhésif selon la revendication 19, pour une utilisation pour la fermeture de plaies.

21. Kit ou adhésif selon la revendication 19,
(i) pour l'utilisation en vue de l'obtention d'un champ opératoire exempt de sang, ou
(ii) pour l'utilisation en vue de la jointure d'anastomoses et de patchs sur des vaisseaux, respectivement des organes creux, ou
(iii) pour l'utilisation dans la cicatrisation de plaies chroniques, ou
(iv) pour l'utilisation dans la désinfection de plaies pour des plaies infectées, ou
(v) pour l'utilisation pour le collage d'organes, de tissus ou d'os, ainsi que d'os avec des implants.

22. Kit ou adhésif selon la revendication 19, comprenant un granulat de zéolithe tel que défini dans l'une quelconque des revendications 7 à 13, pour une utilisation en tant qu'hémostatique.
